# EUROPEAN PATENT SPECIFICATION

(11) **EP 4 433 134 B1**
(45) Date of publication and mention of the grant of the patent: **23.09.2026**
(21) Application number: 22822767.4
(22) Date of filing: 15.11.2022
(51) Int. Cl.: A61M 39/06, A61M 25/02, A61M 25/00, A61M 25/06

(54) **EXPANDABLE SHEATH GASKET TO PROVIDE HOMEOSTASIS**
AUSDEHNBARE HÜLSENDICHTUNG ZUR HOMÖOSTASEBEREITSTELLUNG
JOINT D'ÉTANCHÉITÉ DE GAINE EXTENSIBLE POUR FOURNIR UNE HOMÉOSTASIE

(30) Priority: 17.11.2021 US 202163280251 P; 07.11.2022 US 202263423468 P
(43) Date of publication of application: 25.09.2024
(73) Proprietor: Edwards Lifesciences Corporation, Irvine, CA 92614 (US)
(72) Inventor: RUIZ, David, Guadalupe, Corona, CA 92882 (US); SHWED, Lynne, Borowski, Irvine, CA 92614 (US); FINE, Maxwell, Harrison, Costa Mesa, CA 92627 (US)
(74) Representative: Eisenführ Speiser
(86) International application number: PCT/US2022/049968
(87) International publication number: WO 2023/091413

(56) References cited:
- WO-A1-2014/031860
- WO-A1-2014/123381
- WO-A1-2015/103423
- WO-A1-2018/236953
- WO-A1-2021/146198
- WO-A1-2021/173745
- US-A1- 2003 195 472

## Description

### FIELD

The present application is directed to a sheath for use with catheter-based technologies for repairing and/or replacing heart valves, as well as for delivering an implant, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques. Percutaneous interventional medical procedures utilize the large blood vessels of the body reach target destinations rather than surgically opening target site. There are many types of diseases states that can be treated via interventional methods including coronary blockages, valve replacements (TAVR) and brain aneurysms. These techniques involve using wires, catheters, balloons, electrodes and other thin devices to travel down the length of the blood vessels from the access site to the target site. The devices have a proximal end which the clinician controls outside of the body and a distal end inside the body which is responsible for treating the disease state. Percutaneous interventional procedures offer several advantages over open surgical techniques. First, they require smaller incision sites which reduces scarring and bleeding as well as infection risk. Procedures are also less traumatic to the tissue, so recovery times are reduced. Finally, interventional techniques can usually be performed much faster, and with fewer clinicians participating in the procedure, so overall costs are lowered. In some cases, the need for anesthesia is also eliminated, further speeding up the recovery process and reducing risk.

A single procedure typically uses several different guidewires, catheters, and balloons to achieve the desired effect. One at a time, each tool is inserted and then removed from the access site sequentially. For example, a guidewire is used to track to the correct location within the body. Next a balloon may be used to dilate a section of narrowed blood vessel. Last, an implant may be delivered to the target site. Because catheters are frequently inserted and removed, introducer sheaths are used to protect the local anatomy and simplify the procedure.

An introducer sheath can be used to safely introduce a delivery apparatus into a patient's vasculature (e.g., the femoral artery). Introducer sheaths are conduits that seal onto the access site blood vessel to reduce bleeding and trauma to the vessel caused by catheters with rough edges. An introducer sheath generally has an elongated sleeve that is inserted into the vasculature and a housing that contains one or more sealing valves that allow a delivery apparatus to be placed in fluid communication with the vasculature with minimal blood loss. Once the introducer sheath is positioned within the vasculature, the shaft of the delivery apparatus is advanced through the sheath and into the vasculature, carrying the prosthetic device. Expandable introducer sheaths, allow for the dilating of the vessel to be performed by the passing prosthetic device. Expandable introducer sheaths are disclosed in U.S. Patent No. 8,790,387, entitled "Expandable Sheath for Introducing an Endovascular Delivery Device into a Body," U.S. Patent No. 10,639,152, entitled "Expandable Sheath and Methods of Using the Same," U.S. Application No. 14/880,109, entitled "Expandable Sheath," U.S. Application No. 16/407,057, entitled "Expandable Sheath with Elastomeric Cross Sectional Portions," U.S. Patent No. 10,327,896, entitled "Expandable Sheath with Elastomeric Cross Sectional Portions," U.S. Application No. 15/997,587, entitled "Expandable Sheath for Introducing an Endovascular Delivery Device into a Body," U.S. Application No. 16/378,417, entitled "Expandable Sheath".

Conventional methods of accessing a vessel, such as a femoral artery, prior to introducing the delivery system include dilating the vessel using multiple dilators or sheaths that progressively increase in diameter. Accordingly, delivery and/or removal of prosthetic devices and other material to or from a patient still poses a significant risk to the patient. Repeated insertion and vessel dilation can increase the amount of time the procedure takes, as well as the risk of damage to the vessel. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque. Moreover, some procedures, such as a transseptal approach for mitral valve replacement/repair, require prolonged dilation of incisions in heart tissue and a curving/bending of the sheath to access the treatment site, prolonging procedure time and recovering and increasing risk of trauma to vessels and heart tissue.

Accordingly, there remains a need for further improvements in expandable introducer sheath for endovascular systems used to implant valves and other prosthetic devices.

WO 2018/236953 A1 discloses expandable introducer sheaths. The sheaths allow for temporary expansion of a portion of the sheath to accommodate passage of a delivery system for an implant, then return to a non-expanded state after the passage of the device. The sheath includes a foldable inner member having a detached flap structure at its distal tip that facilitates expansion of the sheath lumen to increased diameters, and an elastomeric distal end that reduces push and retrieval forces therethrough. The sheath includes a hemostasis seal on its proximal end to prevent the leakage of blood out of the sheath and prevent ballooning of outer layer of the sheath. WO 2018/236953 A1 discloses an expandable sheath and methods of using the same.

### SUMMARY

The aforementioned objectives are achieved according to the invention by means of an introducer sheath system according to claim 1. Preferred embodiments are defined in the dependent claims.

The following description also includes various references to methods, none of which fall under the invention as claimed but which serve illustrative purposes.

Furthermore in the description references are made to certain non-SI units (inches). These are to be converted to SI units (mm) using the following factor: 1 inch = approx. 25.4 mm.

Examples of the present expandable sheath can minimize trauma to the vessel by allowing for temporary expansion of a portion of the introducer sheath to accommodate a delivery system, followed by a return to the original diameter once the delivery system passes through. Some examples can include a sheath with a smaller profile than that of prior art introducer sheaths. Furthermore, certain examples can reduce the length of time a procedure takes, as well as reduce the risk of a longitudinal or radial vessel tear, or plaque dislodgement because only one sheath is required, rather than several different sizes of sheaths. Examples of the present expandable sheath can require only a single vessel insertion, as opposed to requiring multiple insertions for the dilation of the vessel. Examples of the present expandable sheath can include a movable exterior seal or gasket for providing hemostasis by constricting the sheath and preventing blood from the patient from penetrating between various layers of the sheath, for example the inner layers of the sheath and the outer elastomeric jacket.

One example gasket includes a gasket body having an inner surface and an outer surface opposite and spaced apart from the inner surface, both surfaces extending between a proximal end and a distal end of the gasket body, the inner surface defining a lumen, wherein the gasket is elastically movable between an unexpanded diameter and an expanded diameter, wherein the gasket is biased towards the unexpanded diameter, wherein the gasket body is configured to expand from the unexpanded diameter toward the expanded diameter upon receiving a threshold radial force against the inner surface of the gasket body.

In some examples, the proximal end defines a proximal inner diameter and the distal end defines a distal inner diameter, and wherein the proximal inner diameter and the distal inner diameter are each sized and configured to accept an introducer sheath into the lumen of the gasket.

In some examples, the proximal end defines a proximal outer diameter, and wherein the distal end defines a distal outer diameter, wherein the proximal outer diameter is greater than the distal outer diameter.

In some examples, the distal outer diameter is sized and configured to be at received at least partially into an inner diameter of a blood vessel closure device.

In some examples, the gasket includes a flared surface at the proximal end adjacent the proximal outer diameter.

In some examples, the flared surface is sized and configured to provide a gripping surface.

In some examples, the gasket includes a tapered surface at the distal end adjacent the distal outer diameter.

In some examples, the tapered surface is sized and configured to seat at least partially in a blood vessel.

In some examples, the gasket is constructed from an elastomeric material (e.g., silicone rubber, nitrile rubber, neoprene, santoprene).

In some examples, the gasket is constructed from a material having a durometer ranging from 40A to 60A.

In some examples, an unexpanded inner diameter ranges from 0.210 inches to 0.315 inches, and the expanded diameter ranges from 0.286 inches to 0.430 inches.

Another example of an introducer sheath system includes a gasket as described above and a sheath for delivering a medical device. The sheath includes a sheath body defining a central lumen extending therethrough, the sheath body having a small diameter portion adjacent a distal end of the body and a large diameter portion adjacent a proximal end of the body, the body including an inner layer defining at least a portion of the central lumen of the sheath, and an outer layer extending at least partially around the inner layer. Wherein the sheath is disposed within the lumen of the gasket. Wherein the gasket provides a radially inward force on the outer layer such that the outer layer is sealed against the inner layer proximate the location of the gasket preventing fluid flow therebetween, and wherein the gasket is longitudinally movable along an outer surface of the sheath from the large diameter portion of the sheath to the distal end of the sheath.

In some examples, the sheath further includes a folded portion configured to move between a folded configuration and a less folded configuration during expansion of the sheath.

In some examples, the inner layer includes a folded portion configured to move between a folded configuration and a less folded configuration during local expansion of the sheath.

In some examples, the folded portion includes a first folded region and a second folded region and an overlapping portion extending between the first and second folded regions, wherein the first folded region is configured to move closer to the second folded region to shorten the overlapping portion at a local axial location during application of a radial outward force by passage of the medical device and wherein shortening of the overlapping portion corresponds with a local expansion of the lumen.

In some examples, the first folded region is configured to move further away from the second folded region to lengthen the overlapping portion at the local axial location after removal of the radial outward force and wherein lengthening of the overlapping portion corresponds with a local contraction of the lumen.

In some examples, the first folded region and the second folded region are circumferentially spaced from each other, wherein the overlapping portion extends circumferentially between the first and second folded regions.

In some examples, the inner layer defines a circumferentially continuous layer and the overlapping portion is radially spaced from an outer surface of a non-overlapping portion of the inner layer, wherein the outer layer defines a discontinuous outer layer including an underlying portion radially spacing the overlapping portion away from the outer surface of the non-overlapping portion.

In some examples, the proximal end of the gasket defines a proximal inner diameter and the distal end of the gasket defines a distal inner diameter, wherein the proximal inner diameter and the distal inner diameter are each sized and configured to accept the sheath into the lumen of the gasket.

In some examples, the proximal end defines a proximal outer diameter, and wherein the distal end defines a distal outer diameter, wherein the proximal outer diameter is greater than the distal outer diameter.

In some examples, the distal outer diameter is sized and configured to be at received at least partially into an inner lumen of a blood vessel closure device.

In some examples, the proximal outer diameter defines a flared surface.

In some examples, the flared surface is sized and configured to provide a gripping surface.

In some examples, the distal outer diameter defines a tapered surface.

In some examples, the taper is sized and configured to seat at least partially in a blood vessel.

In a further example an introducer sheath system includes sheath for introducing a prosthetic device includes an inner layer and an outer layer. At least a portion of the sheath can be designed or configured to locally expand from a first diameter to a second diameter as the prosthetic device is pushed through a lumen of the sheath, and then at least partially return to the first diameter once the prosthetic device has passed through. Some examples can additionally include an elastic outer cover disposed about the outer layer. The inner layer can include polytetrafluoroethylene (PTFE), polyimide, polyetheretherketone (PEEK), polyurethane, nylon, polyethylene, polyamide, or combinations thereof. The outer layer can include PTFE, polyimide, PEEK, polyurethane, nylon, polyethylene, polypropylene, polyamide, polyether block amides, polyether block ester copolymer, thermoset silicone, latex, poly-isoprene rubbers, high density polyethylene (HDPE), Tecoflex^{™}, or combinations thereof. In one example, the inner layer can include PTFE and the outer layer can include a combination of HDPE and Tecoflex^{™}. If present, the elastic outer cover can include any suitable materials, such as any suitable heat shrink materials. Examples include Pebax, polyurethane, silicone, and/or polyisoprene.

Disclosed examples of a sheath include a proximal end and a distal end opposite one another. Some examples can include a hemostasis valve at or near the proximal end of the sheath. In some examples, the outer diameter of the sheath decreases along a gradient from the proximal end to the distal end of the sheath. In other examples, the outer diameter of the sheath is substantially constant along at least a majority of the length of the sheath.

One example of a sheath for introducing a prosthetic device into a body can include a continuous inner layer defining a lumen therethrough, the inner layer having a folded portion and a discontinuous outer layer having an overlapping portion and an underlying portion. In some examples, the inner layer can have at least two folded portions. The outer layer can be configured so that the overlapping portion overlaps the underlying portion, wherein at least a portion of the folded portion of the inner tubular layer is positioned between the overlapping and underlying portions. At least a portion of the sheath is configured to expand to accommodate the prosthetic device.

In some examples, at least a portion of the sheath is configured such that a plurality of segments of the sheath each locally expands one at a time from a rest configuration having a first diameter to an expanded configuration having a second diameter that is larger than the first diameter to facilitate passage of the prosthetic device through the lumen of the inner layer. Each segment can have a length defined along the longitudinal axis of the sheath, and each segment of the sheath can be configured to at least partially return to the first diameter once the prosthetic device has passed through. In some examples, when each segment of the sheath is in the expanded configuration, a length of the folded portion corresponding to the length of the segment at least partially unfolds (*e.g.,* by separating and/or straightening). A length of the overlapping portion corresponding to the length of the segment can be configured to move with respect to the underlying portion when each segment of the sheath expands from the rest configuration to the expanded configuration.

An example method of sealing a sheath includes providing a radially expandable gasket that defines a lumen; providing a sheath that includes a sheath body defining a central lumen extending therethrough, a small diameter portion, and a large diameter portion proximal of the small diameter portion; inserting the sheath into the lumen of the gasket; advancing the gasket proximally to the large diameter portion of the sheath and thereby elastically expanding a diameter of the lumen of the gasket; inserting the sheath into a patient's blood vessel; inserting a medical device through the central lumen of the sheath; advancing the gasket distally to a distal position such that the gasket is exterior to the patient's blood vessel and thereby causing the diameter of the lumen of the gasket to elastically retract, thereby sealing the sheath and preventing bloodflow from the patient proximal of the gasket location.

In some examples, the sheath includes a sheath body defining the central lumen of the sheath and extending therethrough, the sheath body having a small diameter portion adjacent a distal end of the body and a large diameter portion adjacent a proximal end of the body, the body including an inner layer defining at least a portion of the central lumen of the sheath, and an outer layer extending at least partially around the inner layer.

In some examples, sealing the sheath includes sealing a gap between the inner layer and outer layer of the sheath.

In some examples, the method further includes inserting the gasket at least partially into a patient's blood vessel.

In some examples, the method further includes: advancing the gasket proximally over the large diameter portion of the sheath; advancing the medical device proximally through the lumen of the sheath; and removing the sheath from the patient's blood vessel.

A further method of sealing a sheath includes: providing a radially expandable gasket that defines a lumen; providing a sheath that includes a sheath body defining a central lumen extending therethrough, a small diameter portion, and a large diameter portion proximal of the small diameter portion; inserting the sheath into the lumen of the gasket; advancing the gasket proximally to the large diameter portion of the sheath and thereby elastically expanding a diameter of the lumen of the gasket; inserting a medical device through the central lumen of the sheath; and advancing the gasket distally to a distal position such that the gasket is exterior to the patient's blood vessel and thereby causing the diameter of the lumen of the gasket to elastically retract, thereby sealing the sheath and preventing bloodflow from the patient proximal of the gasket location.

Another example gasket includes a housing, a gasket body provided at least partially within the housing, the gasket body having an inner surface and an outer surface opposite and spaced apart from the inner surface, both surfaces extending between a proximal end and a distal end of the gasket body, the inner surface defining a lumen, where the lumen is elastically movable between an unexpanded diameter and an expanded diameter, where the lumen is biased towards the unexpanded diameter, where the lumen is configured to expand from the unexpanded diameter toward the expanded diameter upon receiving a threshold radial force against the inner surface of the gasket body.

In some examples, the proximal end defines a proximal inner diameter, and the distal end defines a distal inner diameter, and where the proximal inner diameter and the distal inner diameter are each sized and configured to accept an introducer sheath into the lumen of the gasket.

In some examples, the gasket is constructed from an elastomeric material (e.g., silicone rubber, nitrile rubber, polyisoprene, neoprene, santoprene).

In some examples, the gasket is constructed from a material having a durometer ranging from 40A to 60A.

In some examples, the unexpanded inner diameter ranges from 0.100 inches to 0.450 inches (e.g., from 0.210 inches to 0.315 inches), and the expanded diameter ranges from 0.286 inches to 0.430 inches.

In some examples, the housing includes an opening for receiving the gasket body such that the housing surrounds the outer surface of the gasket body.

In some examples, the gasket body is provided with in the housing such that the housing prevents radial expansion of the outer surface of the gasket body.

In some examples, at least a portion of a proximal end surface and a distal end surface of the gasket body are provided within the housing.

In some examples, the housing is constructed from a more rigid material than the gasket body (e.g., polycarbonate).

In some examples, the housing includes an attachment feature (e.g., for allowing the gasket to be sutured in place at the skin level of the patient).

In some examples, the attachment feature includes an attachment opening extending through the housing.

In some examples, the attachment opening is provided on an arm extending beyond an outer surface of the housing.

In some examples, an outer perimeter of the housing defines an overall diameter greater than an expanded diameter of an introducer sheath (e.g., the outer perimeter of the housing has a diameter ranging from 0.430 inches to 0.700 inches).

In some examples, a distal outer perimeter of the housing is sized and configured to be at received at least partially into an inner diameter of a blood vessel closure device.

In some examples, an outer surface of the housing is sized and configured to provide a gripping surface (e.g., a flared or textured outer surface).

In some examples, at least a portion of the distal end surface of the housing and/or a distal end surface of the gasket body is configured to seat at least partially in or against the blood vessel and/or other patient anatomy (e.g., the housing can include a tapered surface at the distal end).

Another example of an introducer sheath system includes a gasket, a housing and a sheath body. The gasket includes: a housing; a gasket body provided at least partially within the housing, the gasket body having an inner surface and an outer surface opposite and spaced apart from the inner surface, both surfaces extending between a proximal end and a distal end of the gasket body, the inner surface defining a lumen, where the lumen is elastically movable between an unexpanded diameter and an expanded diameter, where the lumen is biased towards the unexpanded diameter, where the lumen is configured to expand from the unexpanded diameter toward the expanded diameter upon receiving a threshold radial force against the inner surface of the gasket body. The sheath for delivering a medical device is provided. The sheath includes a sheath body defining a central lumen extending therethrough, the sheath body having a small diameter portion adjacent a distal end of the sheath body and a large diameter portion adjacent a proximal end of the sheath body, the sheath body including an inner layer defining at least a portion of the central lumen of the sheath, and an outer layer extending at least partially around the inner layer, where the sheath is disposed within the lumen of the gasket, where the gasket provides a radially inward force on the outer layer such that the outer layer is sealed against the inner layer proximate the location of the gasket preventing fluid flow therebetween, and where the gasket is longitudinally movable along an outer surface of the sheath from the large diameter portion of the sheath to the distal end of the sheath.

In some examples, the sheath further includes a folded portion configured to move between a folded configuration and a less folded configuration during expansion of the sheath.

In some examples, the inner layer includes a folded portion configured to move between a folded configuration and a less folded configuration during local expansion of the sheath.

In some examples, the folded portion comprises a first folded region and a second folded region and an overlapping portion extending between the first and second folded regions, and first folded region is configured to move closer to the second folded region to shorten the overlapping portion at a local axial location during application of a radial outward force by passage of the medical device and wherein shortening of the overlapping portion corresponds with a local expansion of the lumen.

In some examples, the first folded region is configured to move further away from the second folded region to lengthen the overlapping portion at the local axial location after removal of the radial outward force and wherein lengthening of the overlapping portion corresponds with a local contraction of the lumen.

In some examples, the first folded region and the second folded region are circumferentially spaced from each other, and the overlapping portion extends circumferentially between the first and second folded regions.

In some examples, the inner layer defines a circumferentially continuous layer and the overlapping portion is radially spaced from an outer surface of a non-overlapping portion of the inner layer, and the outer layer defines a discontinuous outer layer including an underlying portion radially spacing the overlapping portion away from the outer surface of the non-overlapping portion.

In some examples, the inner layer defines a circumferentially continuous layer and the overlapping portion is radially spaced from an outer surface of a non-overlapping portion of the inner layer, a discontinuous intermediate layer including an underlying portion radially spacing the overlapping portion away from the outer surface of the non-overlapping portion, and outer layer defines an elastic outer jacket extending over both the inner and intermediate layer.

In some examples, the proximal end of the gasket defines a proximal inner diameter and the distal end of the gasket defines a distal inner diameter, and the proximal inner diameter and the distal inner diameter are each sized and configured to accept the sheath into the lumen of the gasket.

In some examples, a distal outer perimeter of the housing is sized and configured to be at received at least partially into an inner lumen of a blood vessel closure device.

In some examples, an outer surface of the housing is sized and configured to provide a gripping surface (e.g., a flared or textured outer surface).

In some examples, at least a portion of the distal end surface of the housing and/or a distal end surface of the gasket body is configured to seat at least partially in blood vessel (e.g., the housing and/or the gasket body can include a tapered surface at the distal end).

A further method of sealing a sheath includes: providing a radially expandable gasket that defines a lumen; providing a sheath that comprises a sheath body defining a central lumen extending therethrough, a small diameter portion, and a large diameter portion proximal of the small diameter portion; inserting the sheath into the lumen of the gasket; advancing the gasket proximally to the large diameter portion of the sheath and thereby elastically expanding a diameter of the lumen of the gasket; inserting the sheath into a patient's blood vessel; inserting a medical device through the central lumen of the sheath; and advancing the gasket distally to a distal position such that the gasket is exterior to the patient's blood vessel and thereby causing the diameter of the lumen of the gasket to elastically retract, thereby sealing the sheath and preventing blood flow from the patient proximal of the gasket location; and securing the gasket to the patient.

In some examples, the method further includes the sheath including a sheath body defining the central lumen of the sheath and extending therethrough, the sheath body having the small diameter portion adjacent a distal end of the body and the large diameter portion adjacent a proximal end of the body, the body comprising an inner layer defining at least a portion of the central lumen of the sheath, and an outer layer extending at least partially around the inner layer.

In some examples, the method further includes sealing the sheath comprises sealing a gap between the inner and outer layers of the sheath.

In some examples, the method further includes the gasket includes an attachment feature, where the gasket is secured to the patient at the attachment feature.

In some examples, the method further includes the attachment feature includes an attachment opening, where securing the gasket to the patient includes suturing the gasket to the patient at a skin level of the patient by passing a suture through the attachment opening in the gasket and into/through the patient (e.g., into/through the patient's).

In some examples, the method further includes inserting the gasket at least partially into a patient's blood vessel.

In some examples, the method further includes: removing the gasket from the patient, advancing the gasket proximally; advancing the medical device proximally through the lumen of the sheath; and removing the sheath from the patient's blood vessel.

Another method of sealing a sheath includes: providing a radially expandable gasket that defines a lumen; providing a sheath that comprises a sheath body defining a central lumen extending therethrough, a small diameter portion, and a large diameter portion proximal of the small diameter portion; inserting the sheath into the lumen of the gasket; advancing the gasket proximally to the large diameter portion of the sheath and thereby elastically expanding a diameter of the lumen of the gasket; inserting a medical device through the central lumen of the sheath; advancing the gasket distally to a distal position such that the gasket is exterior to the patient's blood vessel and thereby causing the diameter of the lumen of the gasket to elastically retract, thereby sealing the sheath and preventing blood flow from the patient proximal of the gasket location; removably coupling the gasket to the patient.

The foregoing and other features and advantages of the invention will become more apparent from the following detailed description, which proceeds with reference to the accompanying figures.

### BRIEF DESCRIPTION OF THE DRAWINGS

FIG. 1 is an elevation view of an expandable sheath along with an endovascular delivery apparatus for implanting a prosthetic implant.
FIG. 2 is an elevation view of an expandable sheath including an introducer locking hub, a sheath locking sleeve, and an introducer.
FIG. 3 is an elevation view of the expandable sheath of FIG. 2 along with an endovascular delivery apparatus for implanting a prosthetic implant.
FIG. 4 is a section view of the distal end of the sheath of FIG. 3, taken along line A-A in FIG. 3.
FIG. 5 is a section view of a proximal section of the sheath of FIG. 35, taken along line B-B in FIG. 3.
FIG. 6 is a section view of the sheath of FIG. 3 in a rest (unexpanded) configuration, taken along line C-C in FIG. 3.
FIG. 7 is the section view of the sheath of FIG. 6, in an expanded configuration.
FIG. 8 is a section view of the sheath of FIG. 3 in a rest (unexpanded) configuration including an outer jacket, taken along line A-A in FIG. 3.
FIG. 9 is a section view of the sheath of FIG. 8 in a rest (unexpanded) configuration, taken along line C-C in FIG. 3.
FIG. 10 is a section view of the sheath of FIG. 9, in an expanded configuration.
FIG. 11 is a section view of the sheath of FIG. 9 in a rest (unexpanded) configuration including a lubricant between the outer layer and the outer jacket.
FIG. 12 is a section view of the sheath of FIG. 9 in a rest (unexpanded) configuration including a lubricant and a bonding strip.
FIG. 13 is a bottom perspective view of the sheath of FIG. 12.
FIG. 14 is a bottom perspective view of the sheath of FIG. 12.
FIG. 15 is a top perspective view of the sheath of FIG. 9.
FIG. 16 is a section view of the sheath of FIG. 18 in a rest (unexpanded) configuration, taken along line C-C in FIG. 3.
FIG. 17 is an elevation view of an expandable outer jacket according to another example.
FIG. 18 is a cross-section view of the expandable sheath of FIG. 17 taken along section line A-A of FIG. 17.
FIG. 19 is a section view of an expandable outer jacket in a rest (unexpanded) configuration, take along section lines B-B of FIG 17.
FIG. 20 is a partial section view of the outer jacket of FIG. 19.
FIG. 21 is a section view of another example outer jacket in a rest (unexpanded) configuration including a single reinforcing member, taken along section lines B-B of FIG. 17.
FIG. 22 is a schematic representation of the sheath extending into the patient's vasculature.
FIG. 23 is a schematic representation of the sheath ballooning in response to blood loss between the sheath layers.
FIG. 24 is an elevation view of a sheath including a gasket disposed about the sheath in a proximal position.
FIG. 25 is an elevation view of the sheath including a gasket disposed about the sheath in a distal position.
FIG. 26 is a schematic representation of an elevation view of the sheath including a gasket having a uniform outer diameter in the proximal position and the distal position.
FIG. 27 is a schematic representation of an elevation view of the sheath including a gasket having a tapered outer diameter in the proximal position and the distal position.
FIG. 28 is perspective view of a gasket and housing.
FIG. 29 is a top view of the gasket and housing of FIG. 28.
FIG. 30 is a cross section view of the gasket of FIG. 29.
FIG. 31 is an elevation view of the sheath including the gasket of FIG. 28.
FIG. 32 is a partial cross section elevation view of the sheath of FIG. 31 positioned in the patient anatomy.

### DETAILED DESCRIPTION

The following description of certain examples of the inventive concepts should not be used to limit the scope of the claims. Other examples, features, aspects, embodiments, and advantages will become apparent to those skilled in the art from the following description. As will be realized, the device and/or methods are capable of other different and obvious aspects. Accordingly, the drawings and descriptions should be regarded as illustrative in nature and not restrictive.

For purposes of this description, certain aspects, advantages, and novel features of the aspects of this disclosure are described herein. The described methods, systems, and apparatus should not be construed as limiting in any way. Instead, the present disclosure is directed toward all novel and nonobvious features and aspects of the various disclosed aspects, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present or problems be solved.

Features, integers, characteristics, compounds, chemical moieties, or groups described in conjunction with a particular aspect or example of the present disclosure are to be understood to be applicable to any other aspect or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive. The present disclosure is not restricted to the details of any foregoing aspects. The present disclosure extends to any novel one, or any novel combination, of the features disclosed in this specification (including any accompanying claims, abstract, and drawings), or to any novel one, or any novel combination, of the steps of any method or process so disclosed.

As used in this application and in the claims, the singular forms "a," "an," and "the" include the plural forms unless the context clearly dictates otherwise. Additionally, the term "includes" means "comprises." Further, the terms "coupled" and "associated" generally means electrically, electromagnetically, and/or physically (e.g., mechanically or chemically) coupled or linked and does not exclude the presence of intermediate elements between the coupled or associated items.

The terms "proximal" and "distal" as used herein refer to regions of a sheath, catheter, or delivery assembly. "Proximal" means that region closest to handle of the device, while "distal" means that region farthest away from the handle of the device.

"Axially" or "axial" as used herein refers to a direction along the longitudinal axis of the sheath.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal aspect. "Such as" is not used in a restrictive sense, but for explanatory purposes.

Disclosed examples of an expandable sheath can minimize trauma to the vessel by allowing for temporary expansion of a portion of the introducer sheath to accommodate the delivery system, followed by a return toward the original diameter once the device passes through. Some examples can comprise a sheath with a smaller profile (e.g., a smaller diameter in the rest configuration) than that of prior art introducer sheaths. Furthermore, present examples can reduce the length of time a procedure takes, as well as reduce the risk of a longitudinal or radial vessel tear, or plaque dislodgement because only one sheath is required, rather than several different sizes of sheaths. Examples of the present expandable sheath can avoid the need for multiple insertions for the dilation of the vessel. Such expandable sheaths can be useful for many types of minimally invasive surgery, such as any surgery requiring introduction of an apparatus into a subject's vessel. For example, the sheath can be used to introduce other types of delivery apparatus for placing various types of intraluminal devices (e.g., stents, prosthetic heart valves, stented grafts, etc.) into many types of vascular and non-vascular body lumens (e.g., veins, arteries, esophagus, ducts of the biliary tree, intestine, urethra, fallopian tube, other endocrine or exocrine ducts, etc.).

Disclosed aspects of an expandable sheath can minimize trauma to the vessel by allowing for temporary expansion of a portion of the introducer sheath to accommodate the delivery system, followed by a return to the original diameter once the device passes through. Furthermore, present aspects can reduce the length of time a procedure takes, as well as reduce the risk of a longitudinal or radial vessel tear, or plaque dislodgement because only one sheath is required, rather than several different sizes of sheaths. Aspects of the present expandable sheath can avoid the need for multiple insertions for the dilation of the vessel.

Disclosed herein are elongate introducer sheaths that are particularly suitable for delivery of implants in the form of implantable heart valves, such as balloon-expandable implantable heart valves. Balloon-expandable implantable heart valves are well-known and will not be described in detail here. An example of such an implantable heart valve is described in U.S. Patent No. 5,411,552, and also in U.S. Patent No. 9,393,110. The expandable introducer sheaths disclosed herein may also be used to deliver other types of implantable medical device, such as self-expanding and mechanically expanding implantable heart valves, stents or filters. Beyond transcatheter heart valves, the introducer sheath system can be useful for other types of minimally invasive surgery, such as any surgery requiring introduction of an apparatus into a subject's vessel. For example, the introducer sheath system can be used to introduce other types of delivery apparatus for placing various types of intraluminal devices (e.g., stents, stented grafts, balloon catheters for angioplasty procedures, etc.) into many types of vascular and non-vascular body lumens (e.g., veins, arteries, esophagus, ducts of the biliary tree, intestine, urethra, fallopian tube, other endocrine or exocrine ducts, etc.). The term "implantable" as used herein is broadly defined to mean anything - prosthetic or not - that is delivered to a site within a body. A diagnostic device, for example, may be an implantable diagnostic device.

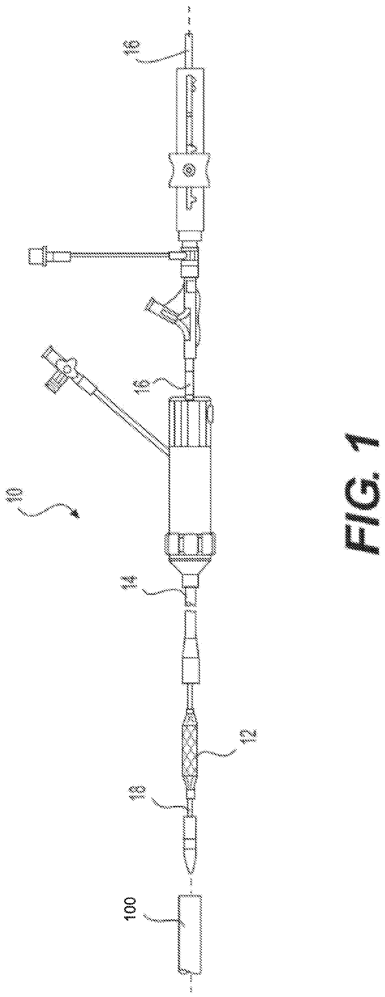

FIG. 1 illustrates an exemplary sheath 100 in use with a representative delivery apparatus 10, for delivering an implant 12, or other type of implantable, to a patient. The delivery apparatus 10 can include a steerable guide catheter 14 (also referred to as a flex catheter) and a balloon catheter 16 extending through the guide catheter 14, and a nose catheter 18 extending through the balloon catheter 16. The guide catheter 14, the balloon catheter 16, and the nose catheter 18 in the illustrated aspect are adapted to slide longitudinally relative to each other to facilitate delivery and positioning of the implant 12 at an implantation site in a patient's body, as described in detail below. The sheath 100 is an elongate, expandable tube that can include a hemostasis valve at the proximal end of the sheath to stop blood leakage.

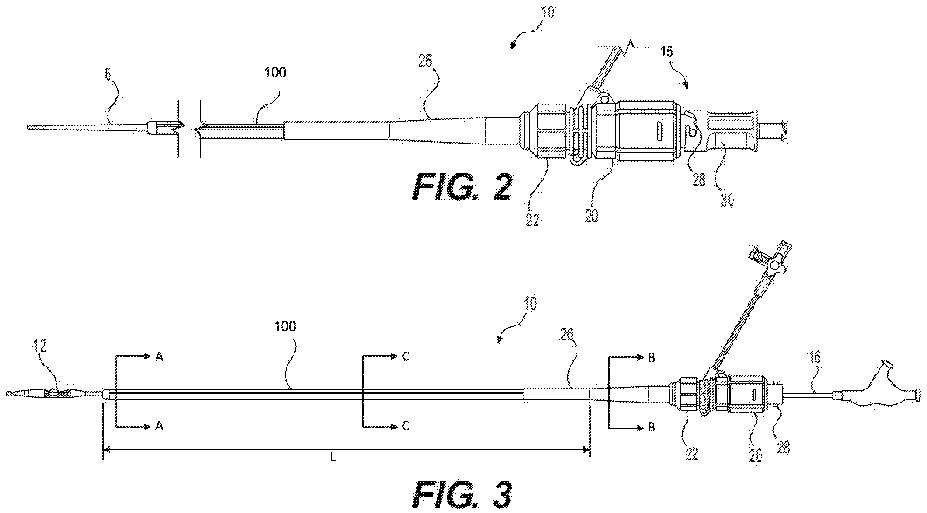

In use, the sheath 100 and an introducer 6 are inserted into a vessel (e.g., the femoral or iliac arteries), passing through the skin of a patient, such that a distal end of the sheath 100 is inserted into the vessel. FIG. 2 illustrates the sheath 100 of FIG. 1 including a sheath locking system 15 which prevents axial and rotational translation of the introducer 6 with respect to the sheath 100. The sheath locking system 15 keeps the introducer 6 fixed with respect to the sheath 100 during insertion without requiring a physician or technician to hold the introducer 6 and the sheath 100 in place at the distal end. The sheath locking system 15 includes a locking sleeve 28 coupled to the sheath 100 via a sheath hub 20 and an introducer locking hub 30 and introducer 6. The locking sleeve 28 engages the introducer locking hub 30 and is moveable between a locked and unlocked position, thereby fixing the position of the introducer 6 and the sheath 100 and preventing movement therebetween during insertion. The sheath locking system 15 keeps the introducer 6 from separating from the sheath 100 and prevents gaps from forming that can cause patient abrasions and unintended fluid flow between the introducer 6 and the sheath 100 during insertion. Example locking systems for use with an expandable delivery sheath are disclosed, for example, in PCT/US2021/050006, filed September 13, 2021 (corresponding U.S. Provisional Application No. 63/077,899 filed September 13, 2020.

As will be described in more detail below, once the sheath 100/introducer 6 is positioned at the delivery site, the introducer 6 is removed from the sheath and the implant 12 is delivered through the sheath 100 and implanted within the patient. FIG. 3 illustrates the sheath of FIGS. 1 and 2 with the introducer 6 removed and the implant 12/balloon catheter 16 passing therethrough.

Example expandable sheaths are also disclosed in U.S. Patent Application No. 12/249,867, filed October 10, 2008 (now U.S. Patent No. 8,690,936), U.S. Patent Application No. 13/312,739, filed December 6, 2011 (now U.S. Patent No. 8,790,387), U.S. Patent Application No. 14/248,120 filed on April 8, 2014 (now U.S. Patent No. 9,301,840), U.S. Patent Application No. 14/324,894, filed July 7, 2014 (now U.S. Patent No. 9,301,841), U.S. Patent Application No. 15/057,953, filed March 1, 2016 (now U.S. Patent No. 9,987,134), U.S. Patent Application No. 15/997,587, filed June 4, 2018, U.S. Patent Application No. 16/149,953, filed on October 2, 2018 (now U.S. Patent No. 10,524,905), U.S. Patent Application No. 16/149,956, filed on October 2, 2018 (now U.S. Patent No. 10,517,720), U.S. Patent Application No. 16/149,960, filed on October 2, 2018 (now U.S. Patent No. 10,524,906, and U.S. Patent Application No. 16/149,969, filed on October 2, 2018 (now U.S. Patent No. 10,524,907).

The sheath 100 can be inserted into the patient's vessel by passing through the skin of patient, such that a soft tip portion at the distal end of the sheath 100 is inserted into the vessel. The sheath 100 can also include a proximal flared end portion to facilitate mating with a sheath hub 20 and catheters mentioned above (e.g., the proximal flared end portion can provide a compression fit over the housing tip and/or the proximal flared end portion can be secured to the sheath hub 20 via a sheath hub cap 22 or other fastening device or by bonding the proximal end of the sheath to the sheath hub 20). The sheath hub 20 provides a housing for necessary valves/seal assemblies and an access point for a secondary lumen (e.g., fluid lumen) in fluid communication with the central lumen of the sheath hub 20. The valves/seals form a seal around the outer surface of the delivery apparatus once inserted through the sheath hub 20. The delivery apparatus can be inserted into and through the sheath 100, allowing the prosthetic device to be advanced through the patient's vasculature and implanted within the patient.

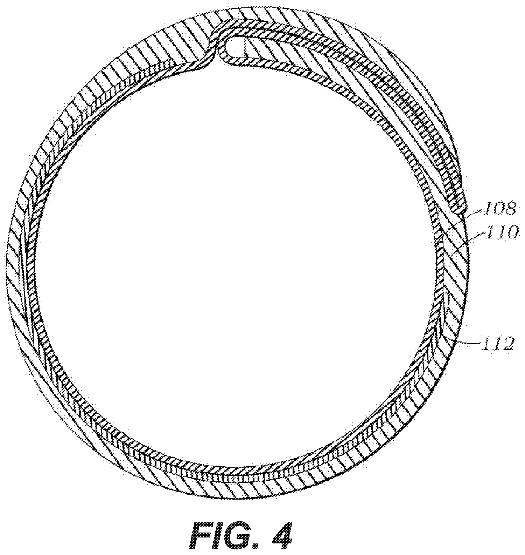

FIG. 4 shows a cross-section view of the sheath 100 taken near the distal end of the sheath 100 taken along section lines A-A (FIG. 3). Sheath 100 can include a plurality of layers. For example, sheath 100 can include an inner layer 108 and an outer layer 110 disposed around the inner layer 108. The sheath 100 can include an outer jacket 140 extending over the outer layer 110. The inner layer 108 can define a lumen through which a delivery apparatus can travel into a patient's vessel in order to deliver, remove, repair, and/or replace a prosthetic device, moving in a direction along the longitudinal axis X. As the prosthetic device passes through the sheath 100, the sheath 100 locally expands from a first, resting diameter to a second, expanded diameter to accommodate the prosthetic device. After the prosthetic device passes through a particular location of the sheath 100, each successive expanded portion or segment of the sheath 100 at least partially returns to the smaller, resting diameter. In this manner, the sheath 100 can be considered self-expanding, in that it does not require use of a balloon, dilator, and/or obturator to expand.

The inner and outer layers 108, 110 can comprise any suitable materials. Suitable materials for the inner layer 108 include polytetrafluoroethylene (PTFE), ethylene tetrafluoroethylene (ETFE), nylon, polyethylene, polyether block amide (e.g., Pebax), and/or combinations thereof. In one specific example the inner layer 108 can comprise a lubricious, low-friction, or hydrophilic material, such as PTFE. Such low coefficient of friction materials can facilitate passage of the prosthetic device through the lumen defined by the inner layer 108. In some examples, the inner layer 108 can have a coefficient of friction of less than about 0.1. Some examples of a sheath 100 can include a lubricious liner on the inner surface of the inner layer 108. Examples of suitable lubricious liners include materials that can further reduce the coefficient of friction of the inner layer 108, such as PTFE, polyethylene, polyvinylidene fluoride, and combinations thereof. Suitable materials for a lubricious liner also include other materials desirably having a coefficient of friction of about 0.1 or less.

Suitable materials for the outer layer 110 include nylon, polyethylene, Pebax, HDPE, polyurethanes (e.g., Tecoflex^{™}), and other medical grade materials. In one example, the outer layer 110 can comprise high density polyethylene (HDPE) and Tecoflex^{™} (or other polyurethane material) extruded as a composite. In some examples, the Tecoflex^{™} can act as an adhesive between the inner layer 108 and the outer layer 110 and may only be present along a portion of the inner surface of the outer layer 110. Other suitable materials for the inner and outer layers are also disclosed in U.S. Patent Application Publication No. 2010/0094392.

Additionally, some examples of a sheath 100 can include an exterior hydrophilic coating on the outer surface of the outer layer 110. Such a hydrophilic coating can facilitate insertion of the sheath 100 into a patient's vessel. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (e.g., PTFE, polyethylene, polyvinylidine fluoride), are also suitable for use with the sheath 100.

The soft tip portion of the sheath 100 can comprise, in some examples, low density polyethylene (LDPE) and can be configured to minimize trauma or damage to the patient's vessels as the sheath is navigated through the vasculature. For example, in some examples, the soft tip portion can be slightly tapered to facilitate passage through the vessels. The soft tip portion can be secured to the distal end of the sheath 100, such as by thermally bonding the soft tip portion to the inner and outer layers of the sheath 100. Such a soft tip portion can be provided with a lower hardness than the other portions of the sheath 100. In some examples, the soft tip can have a Shore hardness from about 25 D to about 40 D. The tip portion is configured to be radially expandable to allow a prosthetic device to pass through the distal opening of the sheath 100. For example, the tip portion can be formed with a weakened portion, such as an axially extending score line or perforated line that is configured to split and allow the tip portion to expand radially when the prosthetic device passes through the tip portion.

FIG. 4 shows a cross-section view of the sheath 100 taken near the distal end of the sheath 100 along section lines A-A (FIG. 3). As shown in 4, the sheath 100 can include at least one radiopaque filler or marker 112, such as a discontinuous, or C-shaped, band positioned near the distal end 104 of the sheath 100. The marker 112 can be associated with the inner and/or outer layers 108, 110 of the sheath 100. For example, as shown in FIG. 4, the marker 112 can be positioned between the inner layer 108 and the outer layer 110. In alternative examples, the marker 112 can be associated with the outer surface of the outer layer 110. In some examples, the marker 112 can be embedded or blended within the inner or outer layers 108, 110.

The C-shaped band can serve as a radiopaque marker or filler, to enable visibility of the sheath 100 under fluoroscopy during use within a patient. The C-shaped band/marker 112 can comprise any suitable radiopaque material, such as barium sulfite, bismuth trioxide, titanium dioxide, bismuth subcarbonate, platinum, iridium, and combinations thereof. In one specific example, the C-shaped band/marker 112 can comprise 90% platinum and 10% iridium. In other examples, the marker 112 need not be a C-shaped band. Other shapes, designs, and configurations are possible. For example, in some examples, the marker 112 can extend around the entire circumference of the sheath 100. In other examples, the marker 112 can comprise a plurality of small markers spaced around the sheath 100.

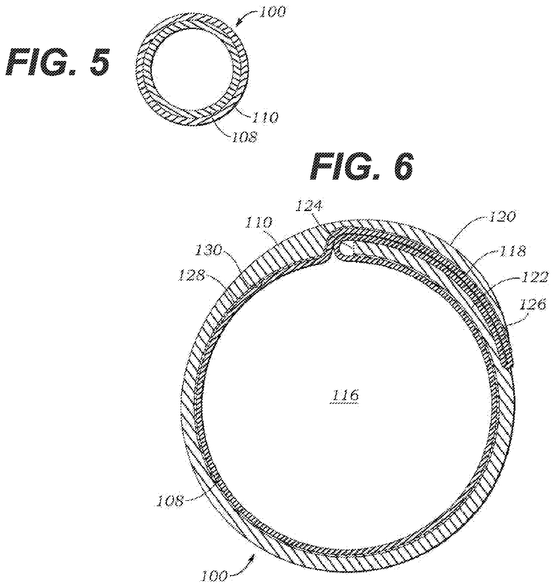

FIGS. 5 and 6 show additional cross sections taken at different points along the sheath 100. FIG. 5 shows a cross-section of a segment of the sheath near the proximal end 106 of the sheath 100, as indicated by line B-B in FIG. 3. The sheath 100 at this location can include inner layer 108 and outer layer 110. At this location, near the proximal end of the sheath, the layers 108, 110 can be substantially tubular, without any slits or folded portions in the layers. By contrast, the layers 108, 110 at different locations along the sheath 100 (e.g., at the point indicated by line C-C in FIG. 3) can have a different configuration.

As shown in FIG. 6, the inner layer 108 can be arranged to form a substantially cylindrical lumen 116 therethrough. Inner layer 108 can include one or more folded portions 118. In the example shown in FIG. 6, inner layer 108 is arranged to have one folded portion 118 that can be positioned on either side of the inner layer 108. The folded portion 118 includes a first fold (e.g., a longitudinally extending fold line) and a second fold and an overlapping portion extending circumferentially therebetween (when the sheath is in an unexpanded configuration). As illustrated in FIG. 6, the folded portion 118 comprises an overlap in a radial direction of at least two thicknesses of the inner layer 108. Inner layer 108 can be continuous, in that there are no breaks, slits, or perforations in inner layer 108. Outer layer 110 can be arranged in an overlapping fashion such that an overlapping portion 120 overlaps at least a part of the folded portion 118 of the inner layer 108. As shown in FIG. 6, the overlapping portion 120 also overlaps an underlying portion 122 of the outer layer 110. The underlying portion 122 can be positioned to underlie both the overlapping portion 120 of the outer layer 110, as well as the folded portion 118 of the inner layer 108. Thus, the outer layer 110 can be discontinuous, in that it includes a slit or a cut in order to form the overlapping and underlying portions 120, 122. In other words, a first edge 124 of the outer layer 110 is spaced apart from a second edge 126 of the outer layer 110 so as not to form a continuous layer.

As shown in FIG. 6, the sheath 100 can also include a thin layer of bonding or adhesive material, also referred to as a tie layer or adhesive layer 128, positioned between the inner and outer layers 108, 110. In one example, the adhesive material can comprise a polyurethane material such as Tecoflex^{™} or etched PTFE tubing. The adhesive material can be positioned on an inner surface 130 of at least a portion of the outer layer 110 so as to provide adhesion between selected portions of the inner and outer layers 108, 110. For example, the outer layer 110 may only include an adhesive layer 128 around the portion of the inner surface 130 that faces the lumen-forming portion of the inner layer 108. In other words, the adhesive layer 128 can be positioned so that it does not contact the folded portion 118 of the inner layer 108 in some examples. In other examples, the adhesive layer 128 can be positioned in different configurations as desired for the particular application. For example, as shown in FIG. 6, the adhesive layer 128 can be positioned along the entire inner surface 130 of the outer layer 110. In an alternative example, the adhesive layer can be applied to the outer surface of the inner layer 108 instead of the inner surface of the outer layer. The adhesive layer 128 can be applied to all or selected portions on the inner layer 108. For example, the adhesive layer 128 can be formed only on the portion of the inner layer that faces the lumen-forming portion of the outer layer and not on the folded portion. The configuration of FIG. 6 allows for radial expansion of the sheath 100 as an outwardly directed radial force is applied from within (e.g., by passing a medical device such as a prosthetic heart valve through the lumen 116). As radial force is applied, the folded portion 118 can at least partially separate, straighten, and/or unfold, and/or the overlapping portion 120 and the underlying portion 122 of the outer layer 110 can slide circumferentially with respect to one another, thereby allowing the diameter of lumen 116 to enlarge.

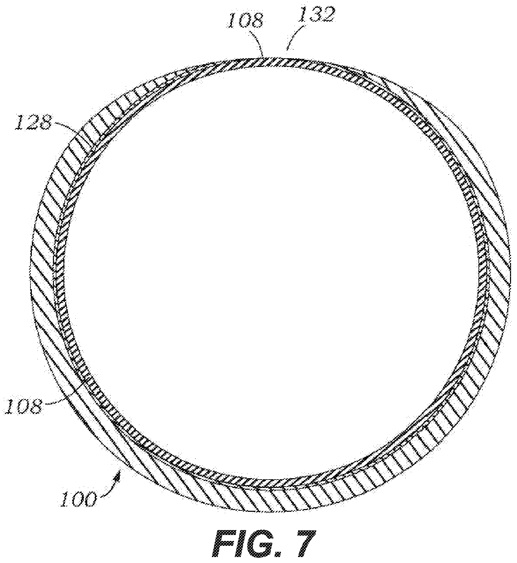

In this manner, the sheath 100 is configured to expand from a resting configuration (FIG. 6) to an expanded configuration shown in FIG. 7. In the expanded configuration, as shown in FIG. 7, an annular gap 132 can form between the longitudinal edges of the overlapping portion 120 and the underlying portion 122 of the outer layer 110. As the sheath 100 expands at a particular location (i.e., locally expands at the location of the passing prosthetic device), the overlapping portion 120 of the outer layer 110 can move circumferentially with respect to the underlying portion 122 as the folded portion 118 of the inner layer 108 unfolds. This movement can be facilitated by the use of a low-friction material for inner layer 108, such as PTFE. Further, the folded portion 118 can at least partially separate and/or unfold to accommodate a medical device having a diameter larger than that of lumen 116 in the resting configuration. As shown in FIG. 7, in some examples, the folded portion of the inner layer 108 can completely unfold, so that the inner layer 108 forms a cylindrical tube at the location of the expanded configuration.

The sheath 100 can be configured such that it locally expands at a particular location corresponding to the location of the medical device along the length of the lumen 116, and then locally contracts once the medical device has passed that particular location. Thus, a bulge may be visible, traveling longitudinally along the length of the sheath as a medical device is introduced through the sheath, representing continuous local expansion and contraction as the device travels the length of the sheath 100. In some examples, each segment of the sheath 100 can locally contract after removal of any radial outward force such that it regains the original resting diameter of lumen 116. In some examples, each segment of the sheath 100 can locally contract after removal of any radial outward force such that it at least partially returns to the original resting diameter of lumen 116.

The layers 108, 110 of sheath 100 can be configured as shown in FIG. 6 along at least a portion of the length of the sheath 100. In some examples, the layers 108, 110 can be configured as shown in FIG. 6 along the length L (FIG. 3) extending from a location adjacent the soft tip portion 102 to a location closer to the proximal end 106 of the sheath 100 (e.g., adjacent and/or including the strain relief portion described below). In this matter, the sheath 100 is expandable and contractable only along a portion of the length of the sheath corresponding to length L (which typically corresponds to the section of the sheath inserted into the narrowest section of the patient's vasculature).

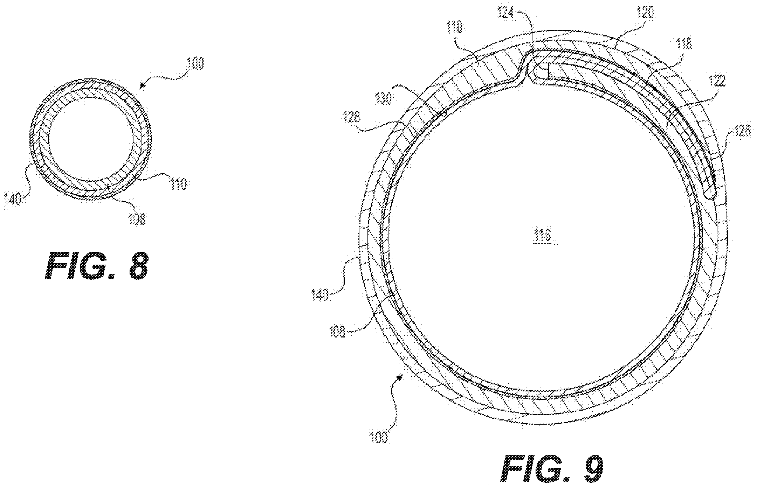

As described generally above, the sheath 100 of FIG. 3 can include an outer jacket 140 extending over the outer layer 110. FIGS. 7 and 8 show additional cross sections taken at different points along the sheath 100. Similar to FIG. 5, FIG. 7 shows a cross-section of a segment of the sheath near the proximal end of the sheath 100, as indicated by line B-B in FIG. 3. The sheath 100 at this location can include inner layer 108, outer layer 110, adhesive layer 128, and an outer jacket 140. At this location, near the proximal end of the sheath, the layers 108, 110 and outer jacket 140 can be substantially tubular, without any slits or folded portions in the layers. By contrast, the layers 108, 110 at different locations along the sheath 100 (e.g., at the point indicated by line C-C in FIG. 3) can have a different configuration, while the outer jacket 140 maintains a substantially tubular shape, without slits or folds.

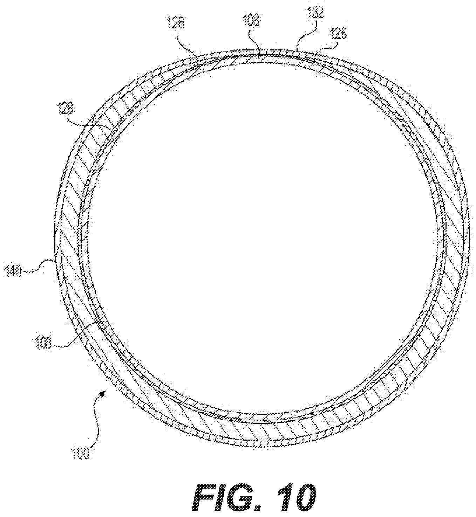

As shown in FIG. 9, and described above with respect to FIG. 6, the inner layer 108 can be arranged to form a substantially cylindrical lumen 116 extending therethrough. The inner layer 108 can include one or more folded portions 118. The outer layer 110 can be arranged in an overlapping fashion such that an overlapping portion 120 overlaps at least a part of the folded portion 118 of the inner layer 108 as well as the underlying portion 122 (positioned to underlie the folded portion 118 of the inner layer 108) when the sheath is unexpanded. The sheath 100 is configured to locally expand from an unexpanded configuration in which the lumen 116 has a first diameter to an expanded configuration in which the lumen 116 has a second diameter larger than the first diameter. The sheath 100 expands in response to an outwardly directed radial force exerted by a medical device against the inner layer 108 as it passes through the lumen 116. During expansion, the first fold/folded edge moves closer to the second fold/folded edge to shorten the folded portion 118. As shown in FIG. 10, in some examples, the folded portion 118 of the inner layer 108 can completely unfold, so that the inner layer 108 forms a cylindrical tube at the location of the expanded configuration. When the sheath is expanded, a portion of the inner layer 108 extends through the opening/gap provided in the outer layer 110, where the opening is formed by the longitudinally extending edge of the overlapping portion 120 and a longitudinally extending edge of the underlying portion 122. As the prosthetic device passes, the sheath 100 then locally contracts at least partially back to the unexpanded configuration.

As described above, the sheath 100 includes an inner layer 108. The inner layer 108 can be surface treated, such as by plasma etching, chemical etching or other suitable methods of surface treatment. By treating the surface of the inner layer 108, the outer surface of the inner layer 108 can have areas with altered surface angles that can provide better adhesion between the inner layer 108 and the outer layer 110. As described above, the inner layer 108 can comprise polytetrafluoroethylene (PTFE), polyimide, polyetheretherketone (PEEK), polyurethane, nylon, polyethylene, polyamide, or combinations thereof. In an example sheath 100, the inner layer 108 is composed of an etched PTFE material. It is contemplated that the inner layer 108 can have a fully etched outer surface or a partially etched outer surface. When partially etched, the unetched portions of the outer surface of the inner layer 108 can extend longitudinally along a length of the inner layer 108 and/or circumferentially around the circumference of the inner layer 108. For example, desired unetched location on the inner layer 108 can be masked or otherwise covered during the etching process to prevent etching at that location. It is also contemplated that the entire outer surface of the inner layer 108 can be etched and the etching removed at the desired locations of unetched surface.

In an example sheath 100, unetched portions are provided along those surfaces of the inner layer 108 that come into contact with the outer surface of the outer layer 110. That is, those portions of the inner layer 108 excluding the tie layer/adhesive layer 128 would not include etching. For example, it is contemplated that etching is not included between the inner surface of the folded portion 118 of the inner layer 108 and the underlying portion 122 of the outer layer 110. By excluding etching on the portions where the inner layer 108 and the outer surface of the outer layer 110 are in direct contact helps to facilitate release of the inner surface of the folded portion 118 and the outer layer 110 during expansion of the sheath 100.

The wall thickness of the inner layer 108 can vary, but in some examples the wall thickness of the inner layer 108 ranges between about 0.002 inches and about 0.006 inches (including about 0.002 inches, about 0.003 inches, about 0.004 inches, about 0.005 inches, about 0.006 inches). In other examples the wall thickness of the inner layer 108 ranges between about 0.003 includes and about 0.005 inches. In a further example, the wall thickness of the inner layer 108 ranges between about 0.0035 inches and about 0.0045 inches (including about 0.0035 inches, about 0.0040 inches, about 0.0045 inches).

As described above, the sheath 100 includes an outer layer 110 exerting a radially inward force on the inner layer 108. In general, the outer layer 110 can comprise a polymeric material. As described above, the outer layer 110 can be comprised of PTFE, polyimide, PEEK, polyurethane, nylon, polyethylene, polypropylene, polyamide, polyether block amides, polyether block ester copolymer, thermoset silicone, latex, poly-isoprene rubbers, high density polyethylene (HDPE), Tecoflex^{™}, or combinations thereof. In an exemplary example, the inner layer 108 can comprise PTFE and the outer layer 110 can comprise a combination of HDPE and Tecoflex^{™}. The outer layer 110 can have a wall thickness ranging between about 0.007 inches and about 0.013 inches (including 0.007 inches, about 0.008 inches, about 0.009 inches, about 0.010 inches, about 0.011 inches, about 0.012 inches, about 0.013 inches). In another example, the outer layer 110 can have a wall thickness ranging between about 0.008 inches and about 0.012 inches. In another example, the outer layer 110 can have a wall thickness ranging between about 0.009 inches and about 0.011 inches.

As described above, the sheath 100 includes an outer jacket 140 that extends over and envelopes the outer layer 110. While the outer layer 110 can be discontinuous, in that it includes a slit or a cut in order to form the overlapping and underlying portions 120, 122 as described above, the outer jacket 140 can include a continuous outer layer covering the inner and outer layers 108, 110.

The outer jacket 140 can be constructed from an elastomeric material such as a soft polymer material having good elasticity, while also being abrasion and tear resistance. Generally, the outer jacket 140 has a relatively low tensile modulus compared to the inner and outer layers 108, 110. Example materials for the outer jacket 140 include, for example, a thermoplastic polyurethane such as Neusoft and Tecoflex^{™} 80A B20. In some examples, the outer jacket 140 comprises an elastomer (e.g., an elastic polymer) with shore hardness (durometer) in the ranging between about 10 and about 95 Shore A. The outer jacket 140 can comprise an elastomer with an elongation at break of ranging between about 40% and about 800% (including about 40%, about 50%, about 60%, about 70%, about 80%, about 90%, about 100%, about 150%, about 200%, about 250%, about 300%, about 350%, about 400%, about 450%, about 500%, about 550%, about 600%, about 650%, about 700%, about 800%). The outer jacket 140 can comprises an elastomer with a wall thickness ranging between about 0.003 inches and about 0.015 inches (including about 0.003 inches, about 0.004 inches, about 0.005 inches, about 0.006 inches, about 0.007 inches, about 0.008 inches, about 0.009 inches, about 0.010 inches, about 0.011 inches, about 0.012 inches, about 0.013 inches, about 0.014 inches, about 0.015 inches). The wall thickness is measured radially between the inner surface of the outer jacket 140 and the outer surface of the outer jacket 140.

In some examples, the outer jacket 140 comprises a single material or combination of materials having a constant thickness along the entire length of the outer jacket 140. In alternative examples, the material composition and/or wall thickness can change along the length of the outer jacket 140. For example, the outer jacket 140 can be provided with one or more segments, where the composition and/or thickness changes from segment to segment. In an example, the Durometer rating of the composition changes along the length of the outer jacket 140 such that segments near the proximal end comprise a stiffer material or combination of materials, while segments near the distal end comprise a softer material or combination of materials. Similarly, the wall thickness of the outer jacket 140 the wall thickness of the outer jacket 140 in segments near the proximal end can be thicker/greater than the wall thickness of the outer jacket 140 near the distal end.

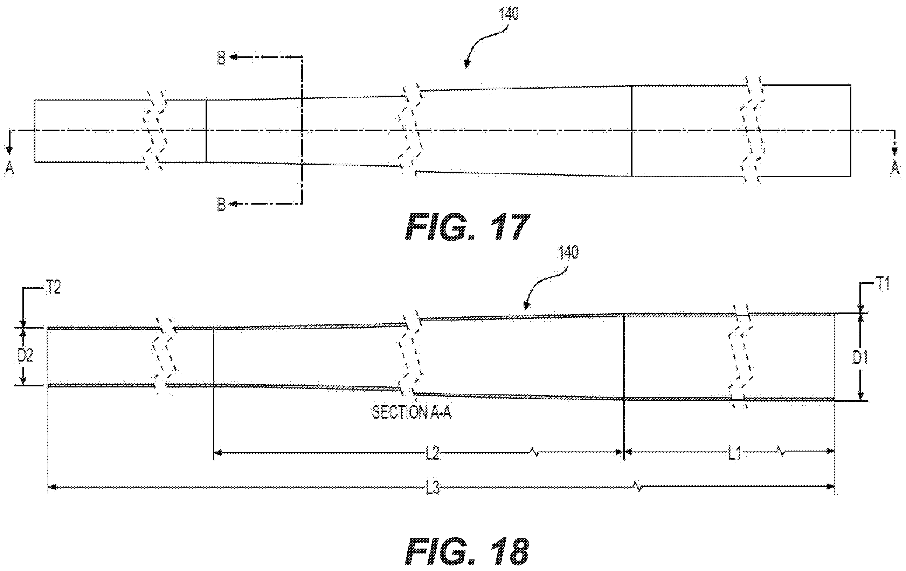

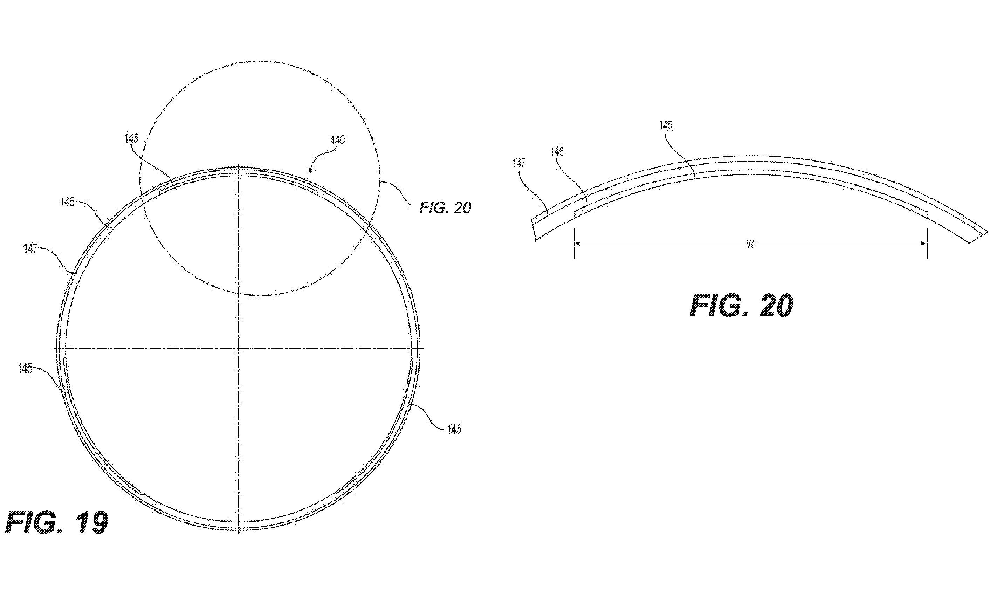

As illustrated in FIG. 19, and described in more detail below with respect to FIGS. 17-21, the outer jacket 140 include one or more axial reinforcing members 145 that extend longitudinally along all or a portion of the outer jacket 140. The reinforcing member 145 provide stiffness and prevent axial bunching of the outer jacket 140 during insertion into the patient's vasculature while not sacrificing the low radial expansion force of the outer jacket 140.

As illustrated in Figure 3, the sheath 100 can include a tapered segment adjacent the flared end portion at the proximal end of the sheath 100. Referred to as a strain relief section 26, the tapered segment and the flared end portion 114 help ease the transition between the smaller diameter portion of the sheath 100 and the sheath hub 20. The thickness and/or composition of the outer jacket 140 can be adjusted to increase the Durometer and/or stiffness along the strain relief section. Because this portion of the sheath 100 is usually outside of the patient's body during procedure, providing the outer jacket 140 with an increased Durometer and/or stiffness along the strain relief section helps to withstand the blood pressure that would otherwise cause the outer jacket 140 to "balloon up" with body fluid/blood. As a result, it allows for a sheath 100 having a relatively stiff proximal end at the point of introducing a delivery apparatus, while still having a relatively soft distal tip at the point of entry into the patient's vessel.

The outer jacket 140 can be bonded to the outer layer 110 to prevent the outer jacket 140 from sliding over the outer layer 110 and "bunching up" in response to the friction forces applied by the surrounding tissue during insertion of the sheath 100 into the patient's vasculature. For example, the outer jacket 140 can be bonded at the proximal end and/or distal end of the outer layer 110. At the proximal and distal ends, the outer jacket 140 can be bonded to the outer layer 110 around the full circumference of the outer layer. At the distal end of the sheath 100, the outer jacket 140 can alternatively be bonded to the inner layer 108. For example, the outer jacket 140 can be bonded to the distal end surface of the inner layer 108.

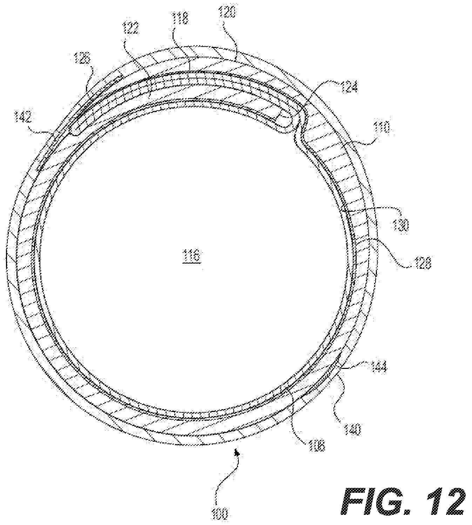

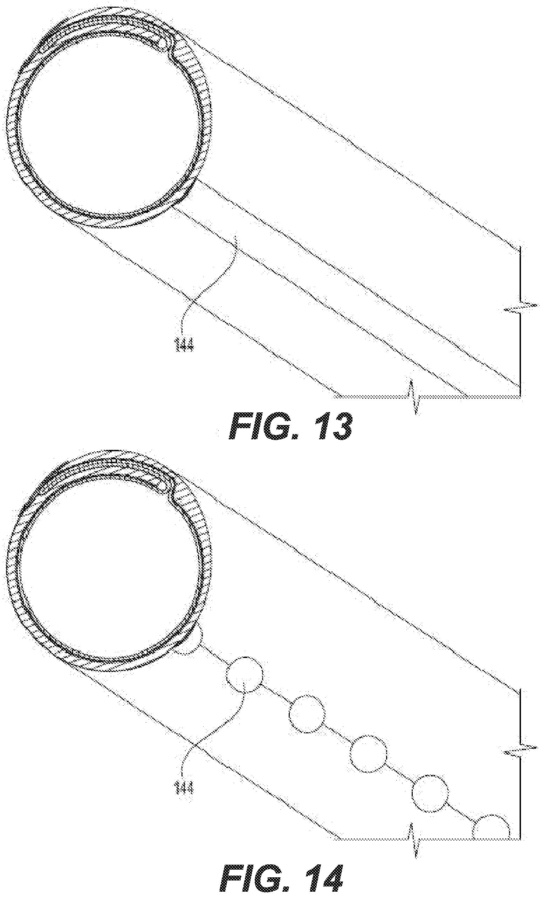

As illustrated in FIG. 12, the outer jacket 140 can be bonded 144 to the outer layer 110 at a circumferential location opposite the folded portion 118 of the inner layer 108. As provided in FIGS. 13-14, the bond 144 can be spot bonds or linear bond lines extending along all or a portion of the outer layer 110. As provided in FIG. 12, the bond 144 line/spot will also have a width, extending circumferentially around the outer layer 110. For example, the bond line can cover about 5° to about 90° of the circumference of the outer layer 110 (including about 5°, about 10°, about 15°, about 20°, about 25°, about 30°, about 35°, about 40°, about 45°, about 50°, about 55°, about 60°, about 65°, about 70°, about 75°, about 80°, about 85°, about 90°).

The outer jacket 140 can be bonded to the outer layer 110 and/or inner layer 108 using any mechanical and/or chemical (e.g., adhesive) fastener known in the art. In an example sheath 100, the outer jacket 140 and the outer layer 110 and/or inner layer 108 may have similar melting temperatures. Accordingly, an example bonding method includes a thermally bonded coupling between the outer jacket 140 the outer layer 110 and/or inner layer 108. For example, the bond between the outer jacket 140 and the outer layer 110 can be achieved by laser welding and/or a heat compression (e.g., using a heat compression jaw), allowing the location of the bond line to be closely controlled.

As shown in FIG. 9, and described above with respect to FIG. 6, and adhesive layer 128 (*e.g.,* a tie layer) is provided between the inner layer 108 and the outer layer 110 to at least partially adhere the inner layer 108 to the outer layer 110. That is, the adhesive layer 128 is selectively provided/located between the inner layer 108 and the outer layer 110 to bond the inner and outer layers 108, 110 at the selected locations of the adhesive layer 128.

As illustrated in FIGS. 9 (and FIG. 6) the adhesive layer 128 is provided on the outer surface of the inner layer 108 and/or the inner surface 130 of the outer layer 110. For example, the adhesive layer 128 can be provided partially or entirely around the outer surface of the inner layer 108. Additionally, or alternatively, the adhesive layer 128 can be provided partially and/or entirely around the inner surface 130 of the outer layer 110. As illustrated in FIG. 9, the adhesive layer 128 extends between the outer layer 110 and the overlapping folded portion 118 of the inner layer 108. That is, the adhesive layer 128 extends between the outer surface of the folded portion 118 of the inner layer 108 and the corresponding inner surface of the overlapping portion 120 of the outer layer 110. As illustrated in FIG. 9, the adhesive layer 128 does not extend between an inner surface of the overlapping folded portion 118 of the inner layer 108 and a corresponding surface of the underlaying portion 122 of the outer surface of the outer layer 110. Excluding the adhesive layer 128 on the portion of the sheath between the inner surface of the folded portion 118 and the underlaying portion 122, facilitates expansion of the sheath and prevents undesirable bonding/sticking between the inner and outer layers 108, 110 at this location.

The adhesive layer 128 can comprise a material having a Shore A hardness (durometer) less than about 90A. For example, the adhesive layer 128 can comprise a thermoplastic polyurethane such as an aliphatic polyether-based thermoplastic polyurethane (TPU). An example TPU includes Tecoflex^{™} 80A. The adhesive layer 128 can also be composed of an aromatic polyether or polyesters based thermoplastic polyurethane such as, for example, Pellethane^{™} 80A. The adhesive layer can also be composed of a polyolefin or polyamide including, for example, a polyolefin (PE, PP, or EVA) modified with maleic anhydride such as an Orevac^{™} resin.

The thickness (wall thickness) of the adhesive layer 128 can vary, but in some examples the wall thickness of the adhesive layer ranges between about 0.002 inches and about 0.005 inches (including about 0.002 inches, about 0.003 inches, about 0.004 inches, about 0.005 inches). In other examples the wall thickness of the adhesive layer 128 ranges between about 0.0025 and about 0.0040 (including about 0.0025 inches, about 0.0030 inches, about 0.0035 inches, about 0.0040 inches). In a further example, the wall thickness of the adhesive layer 128 ranges between about 0.0025 inches and about 0.0035 inches (including about 0.0025 inches, about 0.0030 inches, about 0.0035 inches).

In some examples, the sheath 100 can include a lubricant to reduce friction and facilitate expansion/contraction between the outer layer 110 and the outer jacket 140. The lubricant 142 allows the outer layer 110 and inner layer 108 to unroll easily under the outer jacket 140, ensuring that the hemostasis and atraumatic benefits achieved by the addition of an outer jacket 140 does not comprise the push force performance of the sheath 100. That is, the lubricant 142 reduced the push force necessary to move the prosthetic device through the central lumen 116 of the inner layer 108 during delivery of the prosthetic device and the corresponding local expansion of the sheath 100.

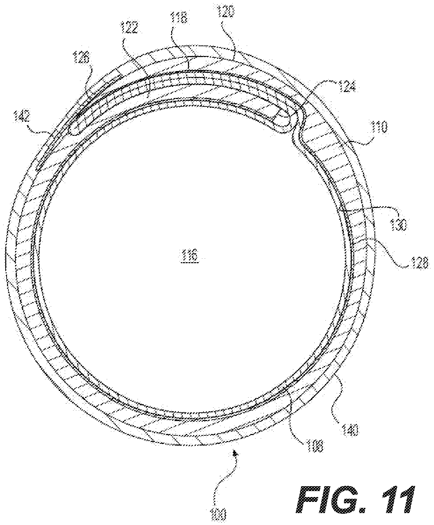

As illustrated in FIG. 11, the lubricant 142 can be selectively applied along an outer surface of the outer layer 110 proximate the longitudinally extending edge 126 of the overlapping portion 120. In some examples, a portion of the folded portion 118 of the inner layer 108 extends beyond the longitudinally extending edge 126 of the overlapping portion 120 and along an outer surface of the outer layer 110. In this example, the lubricant 142 is also provided along the protruding portion the folded portion 118 of the inner layer 108 extending along the outer surface of the outer layer 110 (beyond the edge 126). In this location, the lubricant 142 also reduces friction between the outer jacket 140 and the inner layer 108 during expansion of the sheath 100. As illustrated in FIG. 11, the lubricant 142 extends around the circumference of the outer layer 110 beyond the protruding portion the folded portion 118.

The lubricant is applied as a band (or spot) that extends both circumferentially and longitudinally along the outer layer 110 (and protruding portion of the inner layer 108). In an example sheath 100, the lubricant 142 is applied as a band that extend both circumferentially around the outer layer 110 and longitudinally along a length of the outer layer 110. To prevent migration, the lubricant 142 can be composed of a heat curable material, e.g., a material curable at room temperature. As a result, the material can be applied to a desired location along the outer layer 110 and does not migrate during assembly and/or use of the sheath 100. The lubricant 142 can be composed of a medical grade lubricant, such as silicone. Example lubricants include medical grade curable silicone lubricants including a platinum catalyzed thermal curing silicone lubricant such as NuSilTM MED10-6670 (heat curable), a PTFE lubricant such as Duraglide^{™} (curable at room temperature) and/or CHRISTO-LUBETM.

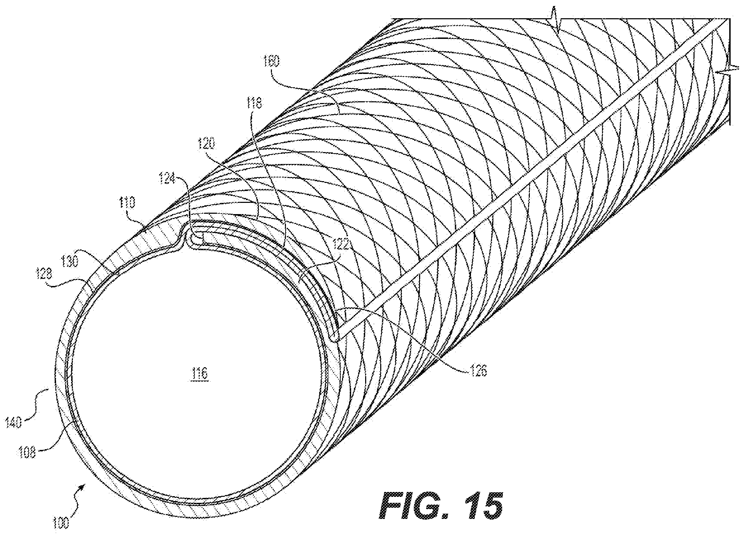

FIG. 15 illustrates an additional example of the sheath 100 of FIG. 3. In this example, the sheath 100 can include a coiled wire 160, or coiled wire mesh, along a length of the sheath 100. The coiled wire 160 provides uniform bending of the sheath and prevents kinking. The coiled wire 160 can be embedded in the outer layer 110. For example, the coiled wire 160 can be co-extruded with the outer layer 110. Alternatively, the coiled wire 160 can be provided between the outer layer and the adhesive layer 128. In another example, the coiled wire 160 is embedded, at least partially, within both the outer layer 110 and the adhesive layer 128. For example, the coiled wire 160 can be provided on an outer surface of the adhesive layer 128 and the outer layer 110 is reflowed over.

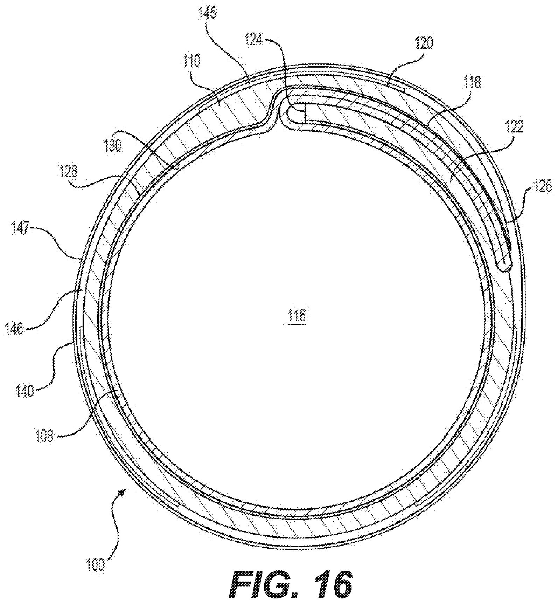

As illustrated in FIG. 16, the coiled wire 160 defines a helical-shaped path around the longitudinal axis of the sheath 100. The example coiled wire 160, includes overlapping helical-shaped path around the longitudinal axis of the sheath 100, resulting in a continuous diamond-shaped pattern along the length of the sheath.

The coiled wire 160 can be comprised of a metal or a polymer wire. For example, the coiled wire 160 can be composed PET, PEEK, stainless steel, and/or nitinol. The coiled wire 160 can be comprised of a flat wire, a round wire, or a combination thereof. The individual wires of the coiled wire 160 can have a diameter/thickness ranging between about 0.002 inches and about 0.008 inches (including about 0.002 inches, about 0.003 inches, about 0.004 inches, about 0.005 inches, about 0.006 inches, about 0.007 inches, about 0.008 inches). In another example, the individual wires of the coiled wire 160 can have a diameter/thickness ranging between about 0.004 inches and about 0.007 inches. In a further example, the individual wires of the coiled wire 160 can have a diameter/thickness of about 0.006 inches. The pitch/distance between adjacent coils of the coiled wire 160 can correspond to the diameter/thickness of the coiled wire. For example, where the diameter/thickness a single coil of the coiled wire is about 0.006 inches, the spacing/pitch between the wire and the next adjacent coiled wire is about 0.006 inches.

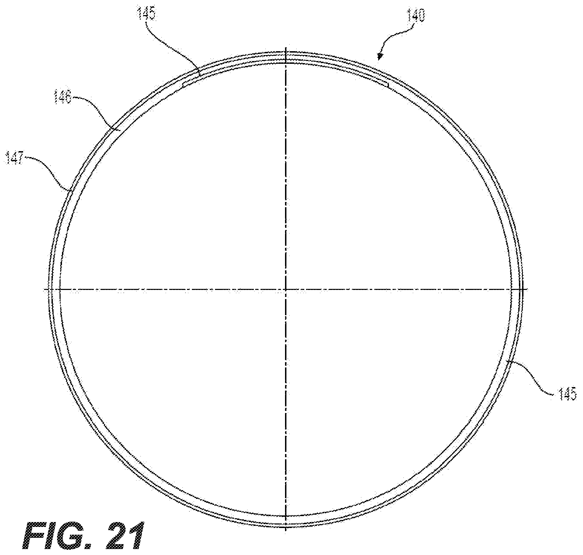

FIGS. 17-21 illustrate side and cross section views of an example outer jacket 140. As described above, the outer jacket 140 includes one or more axial reinforcing members 145 that extend longitudinally along all or a portion of the outer jacket 145. The reinforcing members 145 can be disposed, as individual strips, circumferentially between the inner and outer polymer layers at a predetermined distance from each other. FIG. 19 is a cross section view of the outer jacket 140 taken along section lines B-B in FIG. 17. As provided in FIG. 19, the outer jacket 140 includes three reinforcing members 145. In some examples, the outer jacket 140 includes only one reinforcing member 145 (FIG. 21). In other examples, the outer jacket includes up to eight reinforcing members 145. When more than one reinforcing member 145 is used, the reinforcing members are spaced evenly around the circumference of the outer jacket 140. As further illustrated in FIG. 19, the reinforcing member 145 can have a rectilinear shape (e.g., rectangular) in cross section. However, any other regular or irregular shape is contemplated. The reinforcing member 145 has a finite width that is smaller than the circumference of the outer jacket 140. The total combined width (w) of the reinforcing members 145 can range from 10% to 50% of the circumference of the outer jacket 140. FIG. 20 includes an enlarged partial view of the outer jacket 140 of FIG. 19. As provided in FIG. 20, the circumferential width of the reinforcing members 145 can range from 0.025 inches to 0.100 inches. It is further understood that in the aspects, where the reinforcing member 145 is present as one or more strips disposed circumferentially along the length of the outer jacket 140, the width of the reinforcing member 145 can be the same along the length or it can vary along the length. In aspects, where the reinforcing member 145 width varies along the length of the outer jacket 140, such a reinforcing member 145 can have any of the disclosed above width values. In still further aspects, at least one reinforcing member 145 is configured to provide an axial reinforcement to the outer jacket 140 and as a result to the sheath where the outer jacket 140 can be used. In such exemplary aspects, the at least one reinforcing member 145 can be disposed along the length of the outer jacket 140 or along a portion of the length of the outer jacket 140. In some aspects, the portion of the length of the outer jacket 140 where the at least reinforcing member 145 is disposed is positioned at the distal end and/or proximal end of the outer jacket 140. In yet other aspects, the reinforcing member 145 can also be positioned anywhere along the length of the outer jacket 140.

As illustrated in FIG. 19, the outer jacket 140 includes a two layer construction, inner layer 146 and outer layer 147, where the outer layer provides abrasion resistance (for example, between the sheath and a calcific lesion) and better resistance to hydrophilic coating process, and the inner layer is a more lubricious material (for example, to prevent sticking of the outer jacket against the outer layer of the sheath during expansion) the and provides higher pressure resistance or ballooning resistance and hemostasis. In some aspects, the inner layer 146 (first polymer layer) forms the inner surface of the outer jacket 140 and the outer layer 147 (second polymer layer) forms the outer surface of the outer jacket, the reinforcing members 145 are disposed between the outer surface of the inner layer 146 and the inner surface of the outer layer 147.

In some examples, the inner layer 146 is composed of Pebax or polyurethane having Shore 25D to 35D. In some examples, the inner layer 146 includes a PTFE powder, an optional inorganic filler, and an optional tackiness reducing additive, to lower friction when outer layer 110 of the sheath expanding by sliding against the outer jacket 140. In some examples, the outer layer 147 of the outer jacket 140 is composed of polyurethane or polyurethane/Styrene Block Copolymer (SBC) having Shore A durometer lower than about 60, e.g., Neusoft 597-50A having Shore A hardness of about 55A. In certain examples, the inner layer 146 is constructed from Polyether Block Amide such as Pebax having Shore D durometer less than about 35.

In still further exemplary and unlimiting aspects, the inner layer 146 forms the inner surface of the outer jacket and comprises a first compound composition comprising from greater than 0 wt% to less than 100 wt% of a polymer comprising a polyether block amide, a polyurethane, or a combination thereof based on a total weight of the first compound composition; less than about 65% of an inorganic filler based on a total weight of the first compound composition; and up to about 20 % of a solid lubricant filler based on a total weight of the first compound composition.

Any of the disclosed above inorganic fillers and solid lubricant fillers can be present in any amount as disclosed. For example, the inorganic filler can comprise bismuth oxychloride, barium sulfate, bismuth subcarbonate, calcium carbonate, aluminum trihydrate, barite, kaolin clay, limestone, or any combination thereof. In yet other aspects, the inorganic filler can be present in at least 10 wt %. In still further aspects, the inorganic filler can be present in an amount of less than about 50 wt % based on a total weight of the first compound composition. In yet further aspects, the solid lubricant filler can comprise a PTFE filler. The first compound can also comprise any of the disclosed above additives. For example, the compound can comprise at least one tackiness reducing compound in an amount from about 1 wt% to about 20 wt%.

In still further exemplary aspects, the polymer present in the first compound can have Shore D from about 20D to about 35D, including exemplary values of about 22D, about 25D, about 27D, about 30D, and about 32D.

In yet further aspects, a durometer of the polymer in the inner layer 146 (first polymer layer) composition at a proximal end of the outer jacket 140 can be different from a durometer of the polymer in the inner layer 146 (first polymer layer) composition at a distal end of the outer jacket 140. In still further aspects, the polymer in the first compound can comprise PEBAX^{®}. While in other aspects, the polymer in first compound can comprise polyurethane. In still further aspects, the first compound can also comprise polyamide.

In still further aspects, the outer layer 147 (second polymer layer) can comprise any of the disclosed above polymers. In some aspects, the outer layer 147 can comprise a second compound composition comprising from greater than 0 wt% to 100 wt% of a second polymer comprising polyether block amide, a polyurethane, or a composition thereof. In still further aspects, the outer layer 147 layer can comprise a polyamide. In yet in some other aspects, the second compound can also comprise any of the fillers or additives disclosed above. While in some aspects, the second compound does not comprise the solid lubricant fillers disclosed herein. While in still further aspects, the second compound can comprise a tackiness reducing additive described in this disclosure. In some aspects, the second polymer can be a polyurethane. In still further aspects, the polyurethane is a thermoplastic polyurethane. While in still further aspects, the second polymer can be a blend comprising a polyurethane with a styrene block copolymer. In still further aspects, the blend can further comprise additional polymers and copolymers. For example, ether-based polymers can be present in the blend. In some exemplary and unlimiting aspects, the second polymer can be chosen from commercially available polymers sold under trade name of Neusoft^{™}. In still further aspects, the second polymer can have a Shore A durometer from about 20A to about 75A, including exemplary values of about 25A, about 30A, about 35A, about 40A, about 45A, about 50A, about 55A, about 60A, about 65 A, and about 70A. In yet further aspects, the second polymer can have a Shore A durometer of less than 60A. In some exemplary aspects, the second polymer can be Neusoft^{™} 597-50A.

As provided in FIG. 20, the inner layer 146 is thicker than the outer layer 147. In general, the inner layer has a thickness ranging from 0.0010 inches to 0.0025 inches. In some examples, the inner layer has a thickness of about 0.00154 inches. The outer layer has a thickness ranging from 0.002 inches to 0.004 inches. In some examples, the outer layer has a thickness of about 0.003 inches. In still further aspects, the thickness of the inner layer 146 (first polymer layer) can be from about 1 mil to about 5 mils. In still further aspects, the thickness of the outer layer 147 (second polymer layer) can be from about 1 mil to about 6 mils. In still further aspects, the thickness of the reinforcement member 145 can be any where between about 1 mil to about 6 mils.

As provided in FIG. 19 and 20, the reinforcing members 145 are at least partially embedded in the inner layer 146. In some examples, the thickness of the reinforcing member 145 is less than the thickness of the inner layer 146. For example, as illustrated in FIG. 20, the reinforcing members 145 have a thickness ranging from 0.0005 inches to 0.0015 inches. In some examples, the reinforcing members 145 have a thickness of about 0.001 inches. In an example configuration, the reinforcing members 145 have a thickness of 0.001 inches and the inner layer has a thickness of 0.00154 inches. In another example, not shown, the reinforcing member 145 has a thickness corresponding to the thickness of the inner layer 146. In a further example, the reinforcing member 145 has a thickness greater than the thickness of the inner layer 146. In some examples, the inner layer 146 and the reinforcing member 145 are co-extruded. Similarly, the inner layer 146, reinforcing member 145 and the outer layer 147 are co-extruded with the reinforcing member 145 positioned between the inner and outer layers 146, 147. In other examples, the inner layer 146 is provided over the reinforcing member 145 and the two components are bonded or fused together by at least one of heat or compression.

As described above, the reinforcing members 145 are constructed from a stiffer material than main body portion of the outer jacket 140 (inner layer 146, outer layer 147) and also a material having a low coefficient of friction (e.g., high density polyethylene). In some examples, the reinforcing members 145 are constructed from a polymer compatible to inner layer and outer layer including, for example, high durometer Pebax or polyurethane. The reinforcing member 145 can also be constructed from a material having a Shore D durometer ranging from 45D to 76D.

In some examples, the reinforcing members are constructed from a material excluding PTFE powder or inorganic filler, thereby providing axial compressive/tensile stiffness (from higher durometer material) and friction against axial bunching (without PTFE powder).

In certain examples, the outer layer 147 is constructed from NEUSOFT 597-50, the inner layer 146 is constructed from Pebax 25D including PTFE and BaSO4, and the reinforcing member 145 is constructed from Pebax 35D including PTFE and BaSO4. In further examples, the outer layer 147 is constructed from NEUSOFT 597-50, the inner layer 146 is constructed from Pebax 25D including PTFE and BaSO4, and the reinforcing member 145 is constructed from Pebax 4033. In another example, the outer layer 147 is constructed from NEUSOFT 597-50, the inner layer 146 is constructed from Pebax 25D including PTFE and BaSO4, and the reinforcing member 145 is constructed from Pebax 5533.

In some aspects, the reinforcing member 145 can comprise any of the polymers disclosed herein. In some aspects, the reinforcing member 145 can comprise the first compound disclosed above. Yet in other aspects, the reinforcing member 145 can comprise the second compound disclosed above. While in still further aspects, the reinforcing member 145 can comprise the first compound. Yet in still further aspects, the reinforcing member 145 can comprise any polymers that are known in the art and suitable for the desired application. In some aspects, the reinforcing member 145 can comprise polyether block amide, polyurethane, or a combination thereof. While in still further aspects, the reinforcing member 145 is a polyether block amide, for example PEBAX^{®}. While in still further aspects, the reinforcing member 145 is a polyurethane. In such exemplary aspects, the reinforcing member 145 does not comprise a solid lubricant filler, such as a PTFE. In yet other aspects, the reinforcing member 145 does not comprise an inorganic filler. In still further aspects, the reinforcing member 145 can comprise a polymer comprising PEBAX^{®} or polyurethane having a Shore D durometer between about 45 D to about 90D, including exemplary values of about 50D, about 55D, about 60D, about 65D, about 70D, about 72D, about 75D, about 80D, and about 85D.

In yet further aspects, the reinforcing member 145 can comprise a polyolefin, in still further aspects, the reinforcing member 145 can comprise a polyethylene, a polypropylene, a graft modified polyethylene or polypropylene. In yet further aspects, the reinforcing member 145 can comprise the grafted low-density polyethylene (LDPE), grafted medium density polyethylene, grafted ultra-low-density polyethylene (ULDPE) grafted high density polyethylene (HDPE), grafted heterogeneously branched linear low-density polyethylene (LLDPE), grafted homogeneously branched linear ethylene polymers and substantially linear ethylene polymers, grafted polypropylene, or ethylene vinyl acetate (EVA), or any combination thereof. In such exemplary aspects, a maleic anhydride or an acrylic acid can be used to graft the disclosed above polymers. In still further aspects, the at least one reinforcing member 145 can comprise a maleic anhydride or an acrylic acid grafted low density polyethylene. In yet further aspects, the at least reinforcing member 145 can comprise a maleic anhydride or an acrylic acid grafted polypropylene. In still further aspects, the reinforcing member 145 can comprise a maleic anhydride or an acrylic acid grafted ethylene vinyl acetate. In still further aspects, the reinforcing member 145 can comprise a maleic anhydride grafter polyolefin sold under a trademark of OREVAC^{®}.

In some aspects, the reinforcing member 145 can bond the inner and outer layers 146, 147 and can also assist bonding of the elongated tubing as a whole to an inner member of the sheath. As an example, the reinforcing member 145 is constructed from a thermally bondable strip of tie layer material. Tie layer material is comprised of polyolefin backbone including, for example, LDPE, LLDPE, EVA, HDPE, PP or polyolefin copolymer with grafted functional group such as maleic anhydride or acrylic acid as described below. Tie layer thermally bonds to the polyolefin-like HDPE inner layer 146 and is extruded as a strip in polyamide, co-polyamides, or polyurethane layer of the outer jacket 140. Possible tie layer material for thermally bondable tie layer includes OREVAC^{®} Grafted Polyolefins available from Arkema. In some example, maleic anhydride grafted LLDPE (Linear Low Density Polyethylene) Orevac 18300M is used as a strip. In some examples, the thermally bondable strip is fused to inner layer 146 using heat and/or compression.

In still further aspects, the outer jacket 140 as disclosed herein comprising the reinforcement member can exhibit an expansion force of less than about 50 N. In still further aspects, the outer jacket as disclosed herein that comprises the reinforcement member can exhibit a burst pressure greater than about 8 psi.

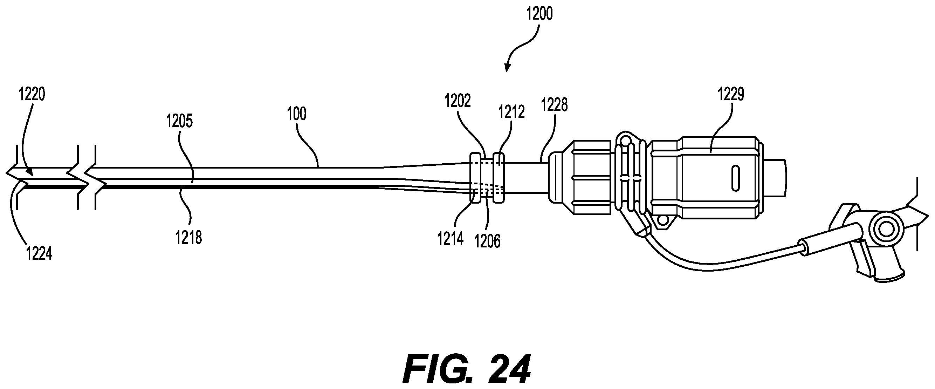

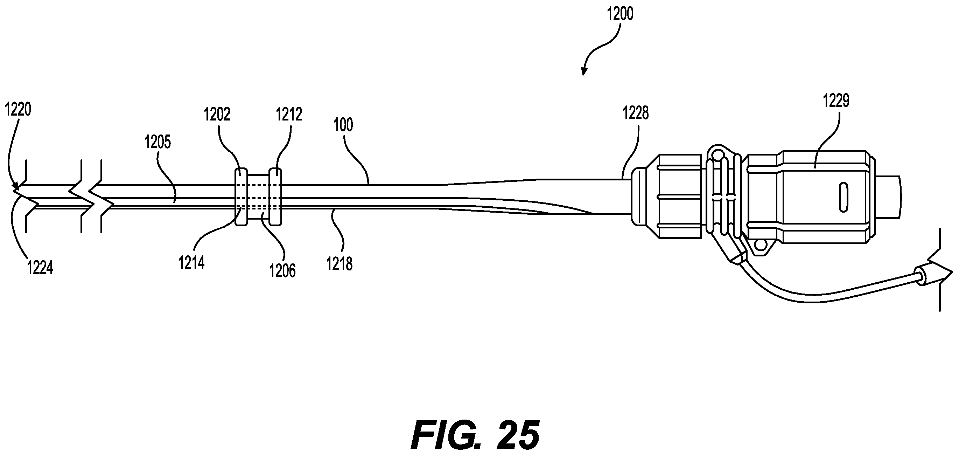

FIGS. 24-27 and 31-32 show an introducer sheath system 1200 that includes a gasket 1202 mounted on the introducer sheath 100. The gasket 1202 is maintains homeostasis between the various layers of the sheath 100 and prevent blood loss through the sheath 100.

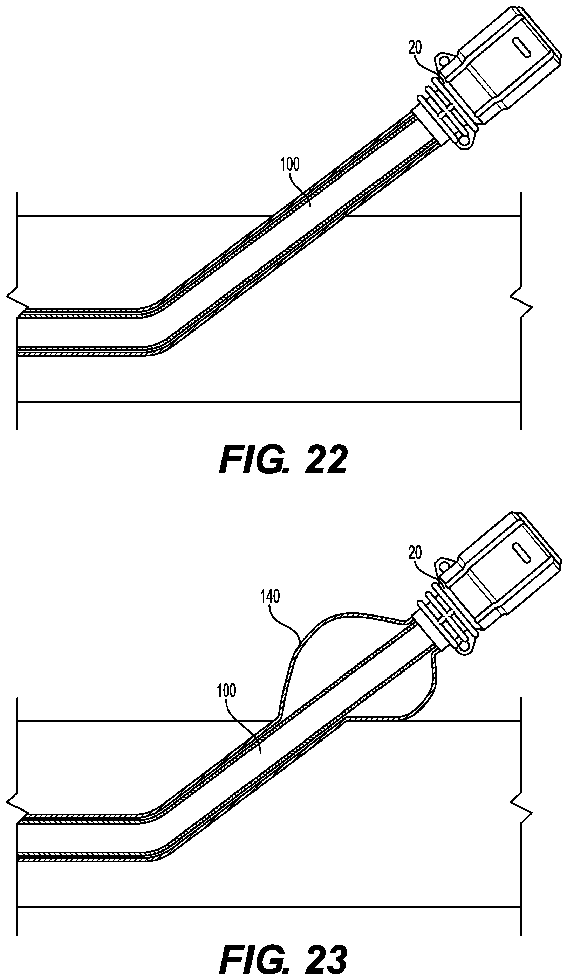

For example, during a for transcatheter aortic valve replacement (TAVR) procedure the sheath 100 is used to provide access to the patient's vasculature (see, for example, FIG. 22). To maintain hemostasis, it is important to ensure that no blood can leak from the patient's vessel through the sheath 100. The sheath hub 20 (FIGS. 2-3) includes valves and/or seals to prevent blood loss through the sheath hub 20, but blood loss can occur through the sheath 100 body itself. Hemostasis may be compromised if blood from the patient's arteriotomy penetrates in between the inner layer 108/outer layer 110 and the outer jacket 140, which can occur after valve expansion causes an intentional break in between the sheath layers at the distal tip.

When this occurs, the outer jacket 140 is the only sheath element that resists the blood pressure to maintain hemostasis. As described above, the outer jacket 140 is made of a compliant material to allow easy expansion of various layers of the sheath 100. As a result, high enough blood pressure may cause the outer jacket 140 to "balloon" which can lead to bursting of the outer jacket and subsequent blood loss (see, for example, FIG. 23). For the portion of the sheath 100 that is within the patient's vessel, ballooning does not occur as the blood pressure within the vessel is the same as the pressure within the sheath 100. However, for the portion of the sheath 100 that is outside the patient, no equalizing pressure is outside of the sheath 100, and ballooning of the outer jacket 140 can occur.

The portion of the sheath 100 that stays outside the patient's body varies and depends on the patient size and anatomy, as well as physician preferences. However, stiffening the outer jacket 140 material to prevent ballooning results in an increase of the force required to expand the various layer of the sheath 100, which in turn increases the amount of push force needed to advance the delivery system through the sheath 100, making the procedure more difficult for the clinician.

As described herein, a movable gasket 1202 is provided to prevent the outer jacket 140 ballooning on the portion of the sheath 100 that remains outside the patient's body during the procedure, while not adversely affecting the force to expand the sheath 100.

The gasket 1202 is provides a movable elastomeric seal that is placed over the outer diameter of the sheath 100 to constrict the outer jacket 140 of the sheath 100 over the delivery apparatus 10 (e.g., passing implant 12 and/or catheter) and/or the various layers of the sheath 100 (e.g., inner and outer layers 108, 110), preventing blood flow proximally of the gasket 1202 and subsequent ballooning of the sheath 100, independent of the length of the sheath 100 inserted into the patient.

The gasket 1202 includes a gasket body 1206, a proximal end 1212, and a distal end 1214. The gasket body 1206 has an inner surface 1208 and an outer surface 1210 opposite and spaced apart from the inner surface 1208. The inner surface 1208 and the outer surface 1210 that each extend between the proximal end 1212 and the distal end 1214 of the gasket body 1206. The inner surface 1208 of the gasket body 1206 defines a lumen 1216 having a uniform diameter therethrough, which provides a radially inward force resulting in a consistent sealing surface along the axial length of the inner surface 1208 of the gasket 1202. The inner surface 1208 has an inner diameter, that is sized and configured to accept the introducer sheath 100 into the lumen 1216 of the gasket 1202. The gasket 1202 is expandable to accept a medical device, such as an expandable heart valve, passing through the lumen of the sheath 100 (and the lumen 1216 of the gasket 1202). The gasket 1202 is elastically movable between an unexpanded configuration, with an unexpanded diameter, and an expanded configuration, with a larger expanded diameter, which allows the lumen 1216 to accommodate devices of various sizes therethrough and conform to the size of the sheath 100 when the sheath 100 is disposed inside the lumen 1216 of the gasket 1202. The gasket 1202 is biased towards the unexpanded diameter such that the gasket 1202 maintains the unexpanded diameter until a threshold force is applied to the gasket 1202 to elastically expand the gasket 1202. The gasket body 1206 expands from the unexpanded diameter toward the expanded diameter upon receiving a threshold radial force against the inner surface 1208 of the gasket body 1206 that overcomes an elastic force provided by the gasket body 1206.

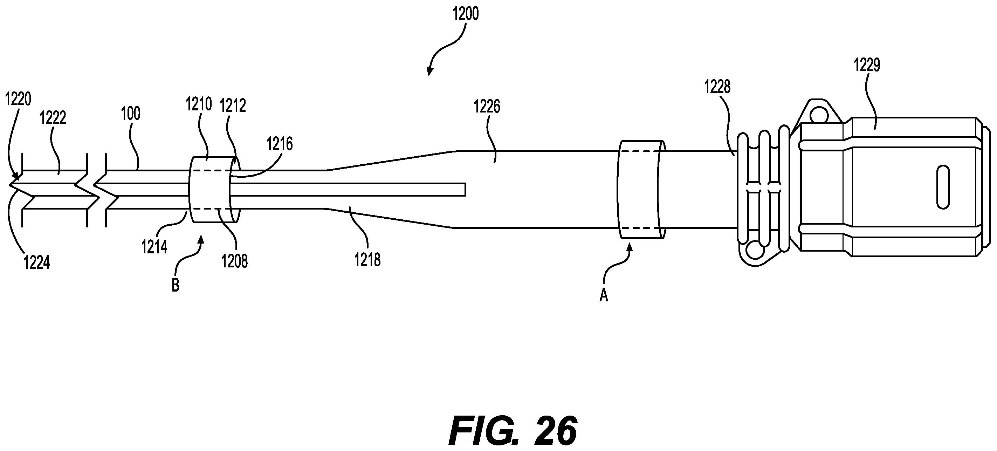

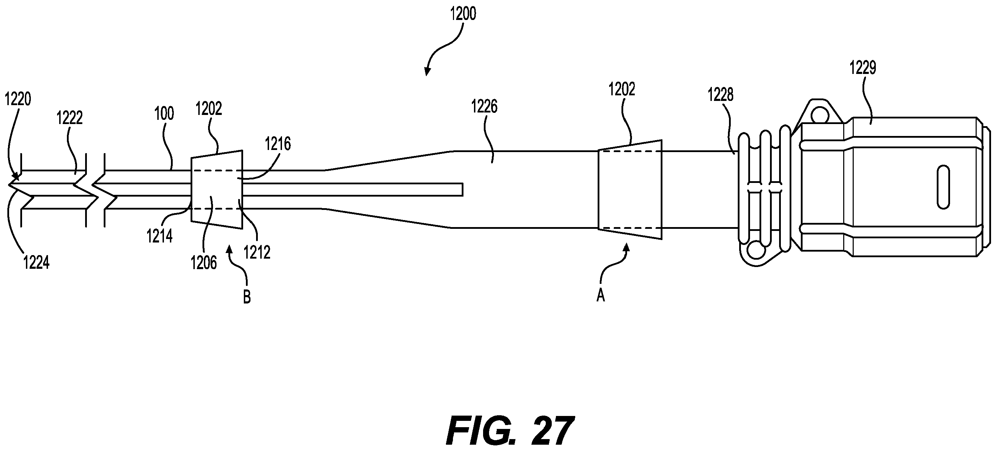

The proximal end 1212 of the outer surface 1210 defines a proximal outer diameter, and the distal end 1214 of the outer surface 1210 defines a distal outer diameter. In some examples the proximal outer diameter and the distal outer diameter are equal and protrude radially outward from a portion of the outer surface 1210 that extends between the proximal end 1212 and the distal end 1214, as shown in FIGS. 24-25. In other examples, the outer diameter at the proximal end 1212 and the distal end 1214 are equal and have a uniform diameter with the outer surface 1210 that extends between the proximal end 1212 and the distal end 1214. For example, FIG. 26 shows an example where the proximal outer diameter is equal to the distal outer diameter, and each have a uniform diameter with the outer surface 1210 of the gasket 1202 extending therebetween. In other examples, the outer surface 1210 is tapered toward the distal outer diameter such that the distal outer diameter is less than the proximal outer diameter. For example, FIG. 27 shows an example where the proximal diameter is greater than the distal diameter. In the example shown in FIG. 27, the distal outer diameter sized and configured to be received at least partially into and/or against an inner diameter of a blood vessel closure device and/or percutaneous suture, e.g., Perclose ProGlide^{™} by Abbot, Angi-Seal^{™} by Terumo, Prostar XL^{™} by Abbot, MANTA^{™} by Teleflex, and/or combinations thereof. For example, the distal end 1214 of the gasket 1202 can include a tapered outer surface 1210 configured to engage a blood vessel closure device/suture extending around at least a portion of gasket 1202. The taper is also sized to allow the gasket 1202 to seat at least partially in and/or against an opening in the blood vessel. The gasket 1202 further includes a flared surface at the proximal end 1212 adjacent the proximal outer diameter, which is sized and configured to provide a gripping surface. The flared surface provides a gripping surface to move the gasket 1202 proximally and distally along the sheath 100.

The gasket 1202 is made of an elastomeric material such as silicone rubber, nitrile rubber, polyisoprene, neoprene, santoprene or any other elastomer suitable to provide a sealing pressure against a fold of a sheath 100 when the sheath 100/gasket 1202 are both expanded and unexpanded. In some examples, the gasket 1202 is made from a material having a durometer ranging from 40A to 60A. In further examples, the gasket 1202 is made from LIM6050 silicone rubber having a durometer 50A, a modulus of elasticity of 0.22MPa, and stiffness characteristics that allow for 530% elongation.

The gaskets 1202 shown in FIGS 24-32 have an unexpanded inner diameter ranging from 0.100 inches to 0.450 inches, or any other diameter suitable to compress a folded portion of a sheath to occlude fluid flow. In some examples, the gasket 1202 has an unexpanded inner diameter ranging from 0.210 inches to 0.315 inches. In further examples, the gasket 1202 has an unexpanded inner diameter ranging from 0.239 inches to 0.263 inches. For example, the gasket 1202 can have an unexpanded inner diameter ranging of 0.263 inches.

The gasket 1202 has an expanded inner diameter large enough to accommodate an implant passing through the central lumen of the sheath 100. For example, the gasket 1202 has an expanded inner diameter ranging from 0.286 inches to 0.430 inches, or any other expanded diameter suitable to accept a medical device such as an expandable heart valve therethrough. In some examples, the gasket has an expanded inner diameter ranging from 0.345 inches to 0.360 inches. For example, the gasket 1202 can have an expanded diameter of 0.358 inches.

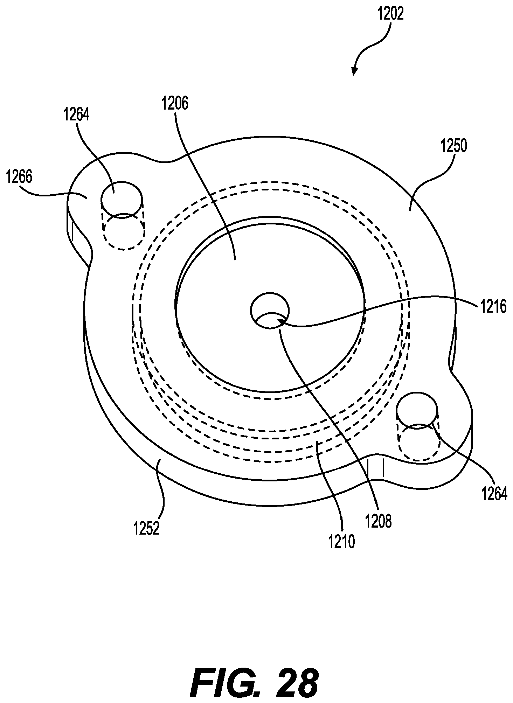

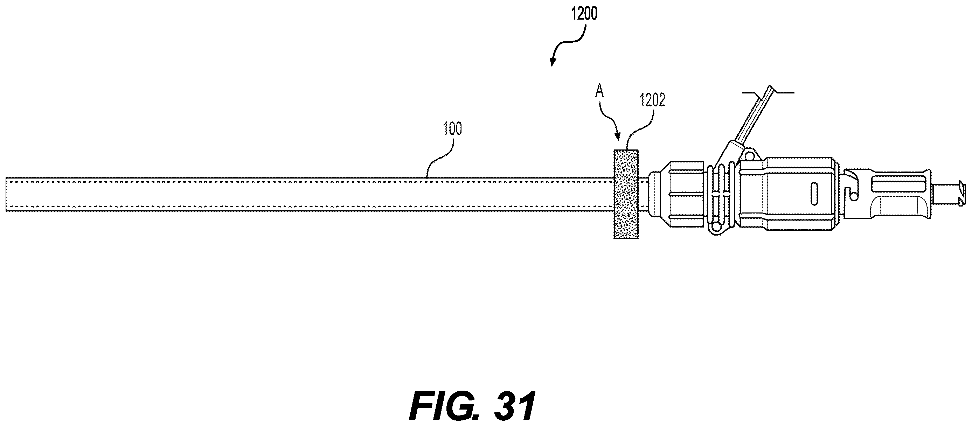

In another example, as illustrated in FIGS. 28-32, the gasket 1202 includes a housing 1250 that extends at least partially around the gasket body 1206. The housing 1250 helps to maintain the position of the gasket body 1206 and allows the assembly to be sutured in place at skin level of the patient. FIG. 31 shows the gasket 1202 with the corresponding housing 1250 mounted on the introducer sheath 100.

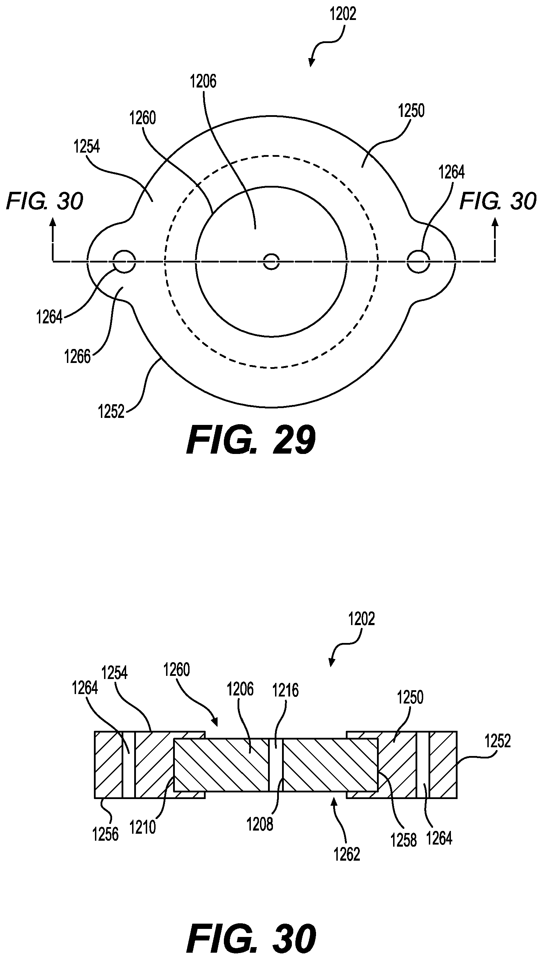

The housing 1250 includes an outer surface extending between proximal and distal end surfaces 1254, 1256 of the housing 1250. While the housing 1250 does not limit radial expansion of the inner surface 1208/lumen 1216 of the gasket body 1206, the housing limits radial expansion of the outer surface 1210 of the gasket body 1206 and prevents the gasket body 1206 from bending. The housing 1250 includes a cavity 1258 for receiving the gasket body 1206 such that the housing 1250 surrounds at least a portion of the outer surface 1210 of the gasket body 1206. As provided in FIG. 30, a portion of the proximal and distal end surfaces of the gasket body 1206 are provided within and/or covered by the housing 1250. As such, the gasket body 1206 is sandwiched between the proximal and distal end surfaces 1254, 1256 of the housing 1250. As provided in FIG. 30, the inner diameter of the cavity 1258 corresponds to the diameter of the outer surface 1210 of the gasket body 1206. As a result, contact/interference between the inner diameter of the cavity 1258 and the outer surface 1210 of the gasket body 1206 limits or otherwise prevents radial expansion of the outer surface 1210 of the gasket body 1206 during delivery of the medical device. Moreover, the housing 1250 is constructed from a more rigid material than the gasket body 1206 (e.g., polycarbonate), further preventing radial expansion of the gasket body 1206 within the housing 1250.

As illustrated in FIG. 28, the housing 1250 further includes top and bottom gasket openings 1260, 1262 provided on the proximal and distal end surfaces 1254, 1256 of the housing 1250. At least a portion of the gasket body 1206 is exposed at the top and bottom gasket openings 1260, 1262. The diameter of the top and bottom gasket openings 1260, 1262 is larger than the expanded diameter of the introducer sheath 100 so that the top and bottom gasket openings 1260, 1262 do not limit expansion of the introducer sheath 100 during delivery of the medical device.

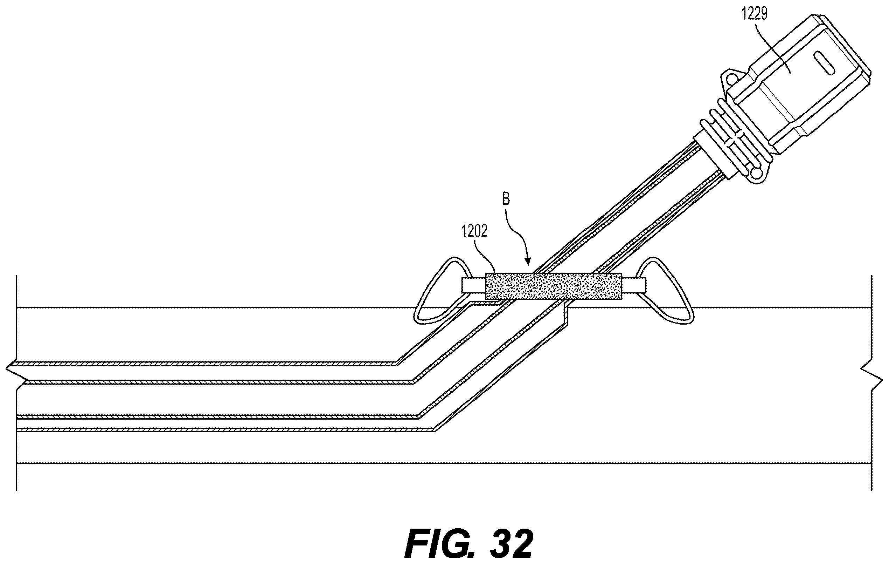

The housing 1250 includes an attachment feature used to couple the gasket 1202 to the patient. As provided in FIG. 28, the attachment feature includes an attachment opening 1264 extending through the housing 1250. As illustrated in FIG. 32, and described in more detail below, a suture passing through the attachment opening 1264 can be used to secure the gasket 1202 to the patient. The attachment opening 1264 is provided on an arm 1266 extending radially from the outer surface 1252 of housing 1250. The housing 1250 can include one attachment opening 1264. In some examples, as illustrated in FIG. 28, the housing 1250 includes two attachment openings 1264 equally spaced radially about the housing 1250, each provided on a corresponding arm 1266 extending radially from the housing 1250. In further examples, the housing 1250 includes two attachment openings 1264 each provided on a corresponding arm 1266 asymmetrically spaced radially about the housing 1250. In still further examples, the housing 1250 includes more than two attachment openings 1264 each provided on a corresponding arm 1266 spaced (symmetrically or asymmetrically) radially about the housing 1250. In some examples, the attachment openings 1264 are provided on/through the housing 1250, and are not included on a radially projecting arm 1266.

The outer surface 1252 and the arms 1266, define the outer perimeter of the housing 1250. In general, the outer perimeter defines an overall diameter greater than an expanded diameter of an introducer sheath 100. For example, the outer perimeter of the housing has a diameter or overall width ranging from 0.430 inches to 0.700 inches. As illustrated in FIGS. 28-30, the outer surface 1252 is a generally cylindrical surface with the arm 1266 projecting therefrom. In some examples the outer surface 1252 has an overall diameter greater than the expanded diameter of the introducer sheath 100. It is contemplated that the outer surface 1252 may define any other regular or irregular shape.

In some examples, the distal outer perimeter and/or the distal end surface 1256 is sized and configured to be received at least partially into and/or against an inner diameter of a blood vessel closure device and/or percutaneous suture. For example, the distal end surface 1256 and/or the distal end of the outer surface 1252 can include a taper or feature configured to engage a blood vessel closure device/suture extending around at least a portion of housing 1250. The taper or feature is also sized to allow the housing 1250 to seat at least partially in and/or against an opening in the blood vessel and/or other patient anatomy.

In addition to the arms 1266, the outer surface 1252 of the housing 1250 is sized and configured to provide a gripping surface for the user. For example, the outer surface 1252 can include a flared or textured surface feature.

Though described in reference to FIGS. 24-32, the gasket 1202 can be used with any introducer sheath according to the various examples described herein. FIGS. 24-32 show an example sheath 100 used in conjunction with the gasket 1202. Sheath 100 includes like features to those described above with reference to FIGS. 1-21. In the examples shown in FIGS 24-32, the introducer sheath 100 includes a sheath body 1218 defining a central lumen 1220 extending therethrough. The sheath body 1218 has a small diameter portion 1222 adjacent a distal end 1224 of the body and a large diameter portion 1226 adjacent a proximal end 1228 of the body. The large diameter portion 1226 functions as a strain relief section as described above, which helps ease the compression transition for a heart valve moving between the small diameter portion 1222 of the sheath 100 and a sheath hub 1229 coupled thereto. The body 1218 further includes an inner layer 1232 defining at least a portion of the central lumen 1220 of the sheath 100, and an outer layer extending at least partially around the inner layer 1232. The sheath 100 includes the folded portion 1205 configured to move between a folded configuration and a less folded configuration during expansion of the sheath 100 as described above.

As shown in FIG 6, and described above, the inner layer of the sheath 100 can include one or more folded portions 118. The folded portion 118 includes a first fold (e.g., a longitudinally extending fold line) and a second fold and an overlapping portion extending circumferentially therebetween (when the sheath is in an unexpanded configuration). The configuration of FIG. 6 allows for radial expansion of the sheath 100 as an outwardly directed radial force is applied from within (e.g., by passing a medical device such as a prosthetic heart valve through the lumen 116). As radial force is applied, the folded portion 118 can at least partially separate, straighten, and/or unfold, and/or the overlapping portion 120 and the underlying portion 122 of the outer layer 110 can slide circumferentially with respect to one another, thereby allowing the diameter of lumen 116 to enlarge.

The sheath 100 is disposed within the lumen 1216 of the gasket 1202. The gasket 1202 can be moved and positioned along the length of the sheath 100 and about the folded portion 1205. The gasket 1202 is radially expandable and contractable to provide an elastic fit against the small diameter portion 1222 of the sheath 100 and the large diameter portion 1226 of the sheath 100 as the sheath 100 is disposed in various longitudinal positions. The gasket 1202 is longitudinally movable along the outer surface of the sheath 100 from the larger diameter portion of the sheath 100 to the distal end 1224 of the sheath 100. The gasket 1202 is elastically coupled to the sheath 100 such that it remains in a fixed position until an axial force acts on it, such as a physician or technician that slides the gasket 1202 along the sheath 100. The gasket 1202 provides a radially inward force on the outer layer such that the outer layer is sealed against the inner layer 1232 proximate the location of the gasket 1202. The gasket 1202 elastically compresses the sheath 100 such that the folded portion 1205 compresses and prevents fluid from passing proximally therethrough.

Methods of using the sheath 100 include providing a gasket 1202 on the outer surface of the sheath 100. The gasket 1202 is radially expandable and defines the lumen 1216 as described above. The sheath 100 can be pre-disposed inside the inner lumen 1216 of the gasket 1202, although in some examples, the sheath 100 is inserted into the lumen 1216 of the gasket 1202 by the user. The gasket 1202 is advanced proximally to the large diameter portion 1226 of the sheath 100 (for example, position A in FIGS. 26, 27, 31), which elastically expands the inner diameter of the gasket 1202 and as such expands the lumen 1216 of the gasket 1202 about the sheath 100. This allows the sheath 100 to be inserted without the gasket 1202 interfering. Furthermore, with the movable gasket 1202 at the proximal edge of the sheath 100, the gasket 1202 is provided over the tapered section of the sheath and positioned at the large diameter portion 1226 which does not expand during delivery system and valve insertion. As a result, the use of the gasket 1202 does not increase the push forces needed for implant delivery.

A prosthetic device and delivery system is then advanced into the inner lumen 116 of the inner layer 108/sheath 100. Before the delivery system is passed through the distal tip of the sheath 100, splitting it open, there is no leak path in between the inner layers (inner layer 108, outer layer 110) and outer jacket 140, so ballooning is unlikely. Once the delivery system is passed through the sheath 100, the distal tip opens and blood may propagate back through the sheath 100 (e.g., between the inner layers (inner layer 108, outer layer 110) and outer jacket 140).

The gasket 1202 is advanced distally toward the distal end 1224 of the sheath 100 (for example, position B in FIG. 26, 27, 31). Because the gasket 1202 is not coupled to the sheath 100, the gasket 1202 is able to freely slide along the shaft of the sheath 100. In some examples, as shown in FIG. 32, the gasket 1202 is advanced to a distal position such that the gasket 1202 is directly exterior and/or abutting to the patient's blood vessel (e.g., the opening in the in the patient's blood vessel for receiving the introducer sheath system), this ensures that blood is prevented from passing proximally back into the sheath 100 beyond the edge of the skin. As blood is prevented from passing between the inner layers and the outer jacket 140 outside the body, ballooning of the outer jacket 140 is prevented. This ensures that no deformation of the outer jacket 140 occurs, preventing blood loss and burst of the sheath 100/outer jacket 140 that can sometimes occur during the procedure.

In some examples, the gasket 1202 is inserted at least partially into the opening in the patient's blood vessel such that the distal end 1214 of the gasket 1202 is positioned at least partially inside the blood vessel. The radially inward force provided by the gasket 1202 urges the inner surface/diameter of the lumen 1216 toward to the sheath 100 and the longitudinal axis the of the sheath 100. As a result, the outer jacket 140 and/or outer layer 110 is sealed against the inner layer 108 thereby sealing any openings between the expanded folded portion 1205 and the inner layer 108 and preventing blood from flowing through/into the folded portion 1205 proximal of the gasket 1202 location (i.e., preventing blood from flowing through/into the expanded folded portion 1205 exterior of the patient).

In some examples, the gasket 1202 is moved into position directly exterior and/or abutting the opening in the patient's blood vessel after the prosthetic device/delivery catheter is advanced into the inner lumen 116 of the sheath 100, but before the prosthetic device/delivery catheter is advanced beyond the distal opening of the sheath 100 and into position at the delivery site.

The prosthetic device applies an outwardly directing radial force on the inner layer 108 of the expandable sheath 100. In some examples, the outwardly directed radial force is transmitted through the inner layer 108, the adhesive layer 128 and the outer layer 110. The lumen 116 of the sheath 100 expands at the axial location of the prosthetic device due to the outwardly directed radial force exerted by a prosthetic device against an inner surface of the lumen during advancement. During expansion of the lumen 116 the first fold (folded edge) of the folded portion 118 is moved circumferentially closer to the second fold (folded edge), shortening the overlapping portion of the folded portion 118 that extends circumferentially between the first and second folds, thereby increasing the circumference of the lumen 116. Without placement of the gasket 1202, blood/fluids would flow from the blood vessel into the expanded folded portion 1205, particularly that portion of the expanded folded portion 1205 exterior to the blood vessel. However, inclusion of the gasket 1202 seals the outer jacket 140 against the outer layer 110 and inner layer 108 and prevents blood from flowing proximal of the gasket 1202 between the layers and/or into the expanded folded portion 1205.

As the sheath 100 expands at a particular location (i.e., locally expands at the location of the passing prosthetic device), the overlapping portion 120 of the outer layer 110 can move circumferentially with respect to the underlying portion 122 as the folded portion 118 of the inner layer 108 least partially separate and/or unfold, causing the elongate gap(s) 132 provided in the outer layer 110 to widen/expand. The sheath thereby expands to accommodate a medical device having a diameter larger than that of lumen 116 in the resting (unexpanded) configuration. As shown in FIG. 10 (and FIG. 7), in some examples, the folded portion of the inner layer 108 can completely unfold, so that the inner layer 108 forms a cylindrical tube at the location of the expanded configuration. As illustrated in FIGS. 9 and 10, the elongate gap 132 is generally aligned with the longitudinal axis of the lumen 116 such that during expansion, the unfolded portion of the inner layer 108 expands into the gap 132.

In an unexpanded configuration, the sheath 100 can have an outer diameter less than about 0.030 inches (including less than about 0.029 inches, less than about 0.028 inches, less than about 0.027 inches, less than about 0.026 inches, less than about 0.025 inches, less than about 0.024 inches). Preferably, the unexpanded sheath 100 has an outer diameter ranging between about 0.024 inches and about 0.026 inches. In a fully expanded configuration, the sheath 100 can have an inner diameter greater than 0.040 inches. Preferably, the expanded sheath 100 has an inner diameter ranging between 0.046 inches and 0.054 inches (including about 0.046 inches, about 0.047 inches, about 0.048 inches, about 0.049 inches, about 0.050 inches, about 0.051 inches, about 0.052 inches, about 0.053 inches, about 0.054 inches)

As described above, the inner and outer layers 108, 110 can be bonded together using an adhesive layer 128. The adhesive layer 128 prevents movement, both longitudinal and radial between the inner and outer layers 108, 110. As a result, expansion of the sheath 100 can be limited to only those regions excluding the adhesive layer 128. For example, as illustrated in FIG. 9, because the adhesive layer 128 is not provided between the inner surface of the folded portion 118 and the underlaying portion 122 of the outer layer, expansion of the sheath results in the inner surface of the folded portion extending into the gap 132 created between the first and second edges 124, 126 of the expanded outer layer 110.

Once the prosthetic device has passed through the lumen 116 (or a particular location along the lumen), the lumen 116 of the sheath can at least partially contract back to an unexpanded configuration. Typically, the medical device has a greater outer diameter than the diameter of the sheath in its original configuration. The medical device can be advanced through the expandable sheath towards the implantation site, and the expandable sheath can locally expand to accommodate the medical device as the device passes through. The radial force exerted by the medical device can be sufficient to locally expand the sheath to an expanded diameter (e.g., the expanded configuration) just in the area where the medical device is currently located. Once the medical device passes a particular location of the sheath, the sheath can at least partially contract to the smaller diameter of its original configuration. For example, the outer layer 110 can exert an inwardly directed radial force on the inner layer 108 urging it back to its original folded configuration. Similarly, if a coiled wire 160 was included, the coiled wire can exert an inwardly directed radial force on the outer layer 110 and the inner layer 108 urging them back towards an unexpanded configuration. Likewise, if an outer jacket 140 is included, the outer jacket 140 can exert an inwardly directed radial force on the outer layer 110 and the inner layer 108 urging them back to an unexpanded configuration. The expandable sheath can thus be expanded without the use of inflatable balloons or other dilators.

The prosthetic device can be delivered through the distal end of the sheath 100 to the delivery site within the patient. The prosthetic device can include a self-expanding heart valve or a stent-mounted heart valve. The heart valve can be extended through the distal end of the elongate inner lumen 116 at the delivery site. Once outside the lumen 116, the heart valve can be expanded/implanted, and the sheath 100 and any sutures holding it in place removed.

In some examples, the gasket 1202 is advanced proximally over the large diameter portion 1226 of the sheath 100, and the delivery device is withdrawn proximally through the distal end 1224 of the sheath 100 through the lumen 1220 of the sheath 100 and toward the proximal end 1228 of the sheath 100. The gasket 1202 can be advanced proximally over the large diameter portion 1226 of the sheath 100 before or after the delivery device is advanced proximally over the large portion of the sheath 100.

In another example, when the example gasket 1202 including a housing 1250 (FIGS. 28-32) is used, the gasket 1202 can be coupled to the patient's skin to secure the gasket 1202 in place. After the prosthetic device/delivery system is advanced into the inner lumen 116 of the sheath 100, the gasket 1202 is advanced distally to a position just above the skin level. Using the attachment opening 1264, the gasket 1202 is coupled to the patient's skin. In some examples, the gasket is sutured to the patient's skin at the attachment opening 1264. With the gasket 1202 coupled to the patient's skin, if the depth of the sheath 100 within the patient's vessel needs to be adjusted during the procedure, the clinician is able to move the sheath while the movable seal remains in place. At the conclusion of the procedure, the sutures attaching the gasket 1202 to the skin can be cut, and the sheath 100 and gasket 1202 together can be removed.

Sheaths of the present disclosure can be used with various methods of introducing a prosthetic device into a patient's vasculature. One such method comprises positioning an expandable sheath in a patient's vessel, passing a device through the introducer sheath, which causes a portion of the sheath surrounding the device to expand and accommodate the profile of the device, and automatically retracting the expanded portion of the sheath to its original size after the device has passed through the expanded portion. In some methods, the expandable sheath can be sutured to the patient's skin at the insertion site so that once the sheath is inserted the proper distance within the patient's vasculature, it does not move once the implantable device starts to travel through the sheath.

Disclosed examples of an expandable sheath can be used with other delivery and minimally invasive surgical components, such as an introducer and loader. In one example, the expandable sheath can be flushed to purge any air within the sheath, using, for example, flush port (e.g., flush port extending from the sheath hub 20, FIG. 3). An introducer can be inserted into the expandable sheath and the introducer/sheath combination can be fully inserted into vasculature over a guiding device, such as a 0.35" guidewire. Preferably, the seam formed by the intersection of the folded portion of the inner layer and the overlapping portion of the outer layer can be positioned such it is oriented downward (posterior). Once the sheath and introducer are fully inserted into a patient's vasculature, in some examples, the expandable sheath can be sutured in place at the insertion site. In this manner, the expandable sheath can be substantially prevented from moving once positioned within the patient. The introducer can then be removed and a medical device, such as a transcatheter heart valve can be inserted into the sheath, in some instances using a loader. Such methods can additionally comprise placing the tissue heart valve in a crimped state on the distal end portion of an elongated delivery apparatus, and inserting the elongated delivery device with the crimped valve into and through the expandable sheath. Next, the delivery apparatus can be advanced through the patient's vasculature to the treatment site, where the valve can be implanted.

In some examples, the gasket is manufactured by injection molding.

The description of the present invention has been presented for purposes of illustration and description but is not intended to be exhaustive or limited to the invention in the form disclosed. Many modifications and variations will be apparent to those of ordinary skill in the art without departing from the scope of the invention. The implementation was chosen and described in order to best explain the principles of the invention and the practical application, and to enable others of ordinary skill in the art to understand the invention for various implementations with various modifications as are suited to the particular use contemplated.

## Claims

1. An introducer sheath system (1200) comprising:
a gasket (1202) comprising:
a gasket body (1206) having an inner surface (1208) and an outer surface (1210) opposite and spaced apart from the inner surface (1208), both surfaces (1208, 1210) extending between a proximal end (1212) and a distal end (1214) of the gasket body (1206), the inner surface (1208) defining a lumen (1216), the gasket (1202) elastically movable between an unexpanded diameter and an expanded diameter and biased towards the unexpanded diameter, where the gasket body (1206) is configured to expand from the unexpanded diameter toward the expanded diameter upon receiving a threshold radial force against the inner surface (1208) of the gasket body (1206); and
a sheath (100) for delivering a medical device, the sheath (100) comprising:
a sheath body defining a central lumen (116) extending therethrough, the sheath body having a small diameter portion adjacent a distal end of the sheath body and a large diameter portion adjacent a proximal end of the sheath body, the sheath body comprising an inner layer (108) defining at least a portion of the central lumen of the sheath, and an outer layer (110) extending at least partially around the inner layer (108),
wherein the sheath (100) is disposed within the lumen (1216) of the gasket (1202),
wherein the gasket (1202) provides a radially inward force on the outer layer (110) such that the outer layer (110) is sealed against the inner layer (108) proximate a location of the gasket (1202) preventing fluid flow therebetween, and
wherein the gasket (1202) is longitudinally movable along an outer surface of the sheath (100) from the large diameter portion of the sheath to the distal end of the sheath (100).

2. The sheath system (1200) of claim 1, wherein the sheath (100) further comprises:
a folded portion (118) configured to move between a folded configuration and a less folded configuration during expansion of the sheath (100).

3. The sheath system (1200) of any one of claims 1 to 2, wherein the inner layer (108) includes a folded portion (118) configured to move between a folded configuration and a less folded configuration during local expansion of the sheath (100).

4. The sheath system (1200) of claim 3, wherein the folded portion (118) comprises a first folded region and a second folded region and an overlapping portion (120) extending between the first and second folded regions, and
wherein the first folded region is configured to move closer to the second folded region to shorten the overlapping portion (120) at a local axial location during application of a radial outward force by passage of the medical device and wherein shortening of the overlapping portion (120) corresponds with a local expansion of the lumen (116).

5. The sheath system (1200) of claim 4, wherein the first folded region is configured to move further away from the second folded region to lengthen the overlapping portion (120) at the local axial location after removal of the radial outward force and wherein lengthening of the overlapping portion (120) corresponds with a local contraction of the lumen (116).

6. The sheath system (1200) of any one of claims 4 to 5, wherein the first folded region and the second folded region are circumferentially spaced from each other, and
wherein the overlapping portion (120) extends circumferentially between the first and second folded regions.

7. The sheath system of any one of claims 4 to 6, wherein the inner layer (108) defines a circumferentially continuous layer and the overlapping portion is radially spaced from an outer surface of a non-overlapping portion of the inner layer (108), and
wherein the outer layer (110) defines a discontinuous outer layer (110) including an underlying portion (122) radially spacing the overlapping portion (120) away from the outer surface of the non-overlapping portion.

8. The sheath system (1200) of any one of claims 1 to 7, wherein the proximal end (1212) of the gasket (1202) defines a proximal inner diameter and the distal end (1214) of the gasket (1202) defines a distal inner diameter, and
wherein the proximal inner diameter and the distal inner diameter are each sized and configured to accept the sheath (100) into the lumen (1216) of the gasket (1202).

9. The sheath system (1200) of any one of claims 1 to 8, wherein the proximal end (1212) defines a proximal outer diameter, and wherein the distal end (1214) defines a distal outer diameter, wherein the proximal outer diameter is greater than the distal outer diameter.

10. The sheath system (1200) of claim 9, wherein the distal outer diameter is sized and configured to be at received at least partially into an inner lumen of a blood vessel closure device.

11. The sheath system (1200) of any one of claims 9 to 10, wherein the proximal outer diameter defines a flared surface.

12. The sheath system (1200) of claim 11, wherein the flared surface is sized and configured to provide a gripping surface.

13. The sheath system (1200) of any one of claims 9 to 12, wherein the distal outer diameter defines a tapered surface.

14. The sheath system (1200) of claim 13, wherein the taper is sized and configured to seat at least partially in a blood vessel.

15. The sheath system (1200) of any one of claims 1 to 14, wherein the gasket is made of an elastomeric materials such as silicone rubber, nitrile rubber, polyisoprene, neoprene or santoprene, preferably from LIM6050 silicone rubber.

## Patentansprüche

1. Einführhülsensystem (1200), umfassend:
eine Dichtung (1202), umfassend:
einen Dichtungskörper (1206), der eine inneren Oberfläche (1208) und eine äußeren Oberfläche (1210) aufweist, die der inneren Oberfläche (1208) gegenüberliegt und von dieser beabstandet ist, wobei sich beide Oberflächen (1208, 1210) zwischen einem proximalen Ende (1212) und einem distalen Ende (1214) des Dichtungskörpers (1206) erstrecken, wobei die innere Oberfläche (1208) ein Lumen (1216) definiert, wobei die Dichtung (1202) elastisch zwischen einem nicht expandierten Durchmesser und einem expandierten Durchmesser beweglich und in Richtung des nicht expandierten Durchmessers vorgespannt ist, wobei der Dichtungskörper (1206) ausgebildet ist, von dem nicht expandierten Durchmesser zu dem expandierten Durchmesser hin zu expandieren, wenn eine Schwellenwert-Radialkraft gegen die innere Oberfläche (1208) des Dichtungskörpers (1206) aufgenommen ist; und
eine Hülse (100) zum Zuführen einer medizinischen Vorrichtung, wobei die Hülse (100) umfasst:
einen Hülsenkörper, der ein zentrales Lumen (116) definiert, das sich durch diesen hindurch erstreckt, wobei der Hülsenkörper einen Abschnitt mit kleinem Durchmesser benachbart zu einem distalen Ende des Hülsenkörpers und einen Abschnitt mit großem Durchmesser benachbart zu einem proximalen Ende des Hülsenkörpers aufweist, wobei der Hülsenkörper eine Innenschicht (108) umfasst, die zumindest einen Abschnitt des zentralen Lumens der Hülse definiert, und eine Außenschicht (110), die sich zumindest teilweise um die Innenschicht (108) herum erstreckt,
wobei die Hülse (100) innerhalb des Lumens (1216) der Dichtung (1202) angeordnet ist,
wobei die Dichtung (1202) eine radial nach innen gerichtete Kraft auf die Außenschicht (110) bereitstellt, so dass die Außenschicht (110) gegen die Innenschicht (108) in der Nähe einer Position der Dichtung (1202) abgedichtet ist und einen Fluidfluss dazwischen verhindert, und
wobei die Dichtung (1202) entlang einer äußeren Oberfläche der Hülse (100) von dem Abschnitt mit großem Durchmesser der Hülse zu dem distalen Ende der Hülse (100) longitudinal beweglich ist.

2. Das Hülsensystem (1200) nach Anspruch 1, wobei die Hülse (100) weiter umfasst:
einen gefalteten Abschnitt (118), der ausgebildet ist, sich während der Expansion der Hülse (100) zwischen einer gefalteten Konfiguration und einer weniger gefalteten Konfiguration zu bewegen.

3. Das Hülsensystem (1200) nach einem der Ansprüche 1 bis 2, wobei die Innenschicht (108) einen gefalteten Abschnitt (118) aufweist, der ausgebildet ist, sich während der lokalen Expansion der Hülse (100) zwischen einer gefalteten Konfiguration und einer weniger gefalteten Konfiguration zu bewegen.

4. Das Hülsensystem (1200) nach Anspruch 3, wobei der gefaltete Abschnitt (118) einen ersten Faltbereich und einen zweiten Faltbereich sowie einen Überlappungsabschnitt (120) umfasst, der sich zwischen dem ersten und dem zweiten Faltbereich erstreckt, und
wobei der erste Faltbereich ausgebildet ist, sich näher an den zweiten Faltbereich zu bewegen, um den Überlappungsabschnitt (120) an einer lokalen axialen Position während der Einwirkung einer radial nach außen gerichteten Kraft durch den Durchgang der medizinischen Vorrichtung zu verkürzen, und wobei die Verkürzung des Überlappungsabschnitts (120) mit einer lokalen Expansion des Lumens (116) korrespondiert.

5. Das Hülsensystem (1200) nach Anspruch 4, wobei der erste Faltbereich ausgebildet ist, sich weiter von dem zweiten Faltbereich zu entfernen, um den Überlappungsabschnitt (120) an der lokalen axialen Position nach Entfernung der radial nach außen gerichteten Kraft zu verlängern, und wobei die Verlängerung des Überlappungsabschnitts (120) mit einer lokalen Kontraktion des Lumens (116) korrespondiert.

6. Das Hülsensystem (1200) nach einem der Ansprüche 4 bis 5, wobei der erste Faltbereich und der zweite Faltbereich in Umfangsrichtung voneinander beabstandet sind, und
wobei sich der Überlappungsabschnitt (120) in Umfangsrichtung zwischen dem ersten und dem zweiten Faltbereich erstreckt.

7. Das Hülsensystem nach einem der Ansprüche 4 bis 6, wobei die Innenschicht (108) eine in Umfangsrichtung kontinuierlichen Schicht definiert und der Überlappungsabschnitt radial von einer äußeren Oberfläche eines nicht überlappenden Abschnitts der Innenschicht (108) beabstandet ist, und
wobei die Außenschicht (110) eine diskontinuierliche Außenschicht (110) definiert, die einen darunterliegenden Abschnitt (122) umfasst, der den Überlappungsabschnitt (120) radial von der äußeren Oberfläche des nicht überlappenden Abschnitts beabstandet.

8. Das Hülsensystem (1200) nach einem der Ansprüche 1 bis 7, wobei das proximale Ende (1212) der Dichtung (1202) einen proximalen Innendurchmesser definiert und das distale Ende (1214) der Dichtung (1202) einen distalen Innendurchmesser definiert, und
wobei der proximale Innendurchmesser und der distale Innendurchmesser jeweils bemessen und ausgebildet sind, die Hülse (100) in das Lumen (1216) der Dichtung (1202) aufzunehmen.

9. Das Hülsensystem (1200) nach einem der Ansprüche 1 bis 8, wobei das proximale Ende (1212) einen proximalen Außendurchmesser definiert, und wobei das distale Ende (1214) einen distalen Außendurchmesser definiert, wobei der proximale Außendurchmesser größer ist als der distale Außendurchmesser.

10. Das Hülsensystem (1200) nach Anspruch 9, wobei der distale Außendurchmesser bemessen und ausgebildet ist, zumindest teilweise in ein inneres Lumen einer Blutgefäßverschlussvorrichtung aufgenommen zu sein.

11. Das Hülsensystem (1200) nach einem der Ansprüche 9 bis 10, wobei der proximale Außendurchmesser eine ausgestellte Oberfläche definiert.

12. Das Hülsensystem (1200) nach Anspruch 11, wobei die ausgestellte Oberfläche bemessen und ausgebildet ist, eine Grifffläche bereitzustellen.

13. Das Hülsensystem (1200) nach einem der Ansprüche 9 bis 12, wobei der distale Außendurchmesser eine sich verjüngende Oberfläche definiert.

14. Das Hülsensystem (1200) nach Anspruch 13, wobei die Verjüngung bemessen und ausgebildet ist, zumindest teilweise in einem Blutgefäß zu sitzen.

15. Das Hülsensystem (1200) nach einem der Ansprüche 1 bis 14, wobei die Dichtung aus einem elastomeren Material besteht, wie beispielsweise Silikonkautschuk, Nitrilkautschuk, Polyisopren, Neopren oder Santoprene, vorzugsweise aus LIM6050-Silikonkautschuk.

## Revendications

1. Système de gaine d'introduction (1200) comprenant :
un joint d'étanchéité (1202) comprenant :
un corps (1206) de joint d'étanchéité présentant une surface interne (1208) et une surface externe (1210) opposée et écartée de la surface interne (1208), les deux surfaces (1208, 1210) s'étendant entre une extrémité proximale (1212) et une extrémité distale (1214) du corps (1206) de joint d'étanchéité, la surface interne (1208) définissant une lumière (1216), le joint d'étanchéité (1202) étant élastiquement mobile entre un diamètre non étendu et un diamètre étendu et sollicité vers le diamètre non étendu, le corps (1206) de joint d'étanchéité étant conçu pour s'étendre à partir du diamètre non étendu vers le diamètre étendu lors de la réception d'une force radiale de seuil contre la surface interne (1208) du corps (1206) de joint d'étanchéité ; et
une gaine (100) pour la pose d'un dispositif médical, la gaine (100) comprenant :
un corps de gaine définissant une lumière centrale (116) s'étendant à travers celui-ci, le corps de gaine présentant une partie de petit diamètre adjacente à une extrémité distale du corps de gaine et une partie de grand diamètre adjacente à une extrémité proximale du corps de gaine, le corps de gaine comprenant une couche interne (108) définissant au moins une partie de la lumière centrale de la gaine et une couche externe (110) s'étendant au moins partiellement autour de la couche interne (108),
dans lequel la gaine (100) est disposée à l'intérieur de la lumière (1216) du joint d'étanchéité (1202),
dans lequel le joint d'étanchéité (1202) fournit une force radialement vers l'intérieur sur la couche externe (110) de telle sorte que la couche externe (110) est scellée contre la couche interne (108) à proximité d'un emplacement du joint d'étanchéité (1202) empêchant un écoulement de fluide entre celles-ci et
dans lequel le joint d'étanchéité (1202) est mobile longitudinalement le long d'une surface externe de la gaine (100) à partir de la partie de grand diamètre de la gaine vers l'extrémité distale de la gaine (100).

2. Système de gaine (1200) selon la revendication 1, dans lequel la gaine (100) comprend en outre :
une partie pliée (118) conçue pour se déplacer entre une configuration pliée et une configuration moins pliée pendant l'extension de la gaine (100).

3. Système de gaine (1200) selon l'une quelconque des revendications 1 à 2, dans lequel la couche interne (108) comprend une partie pliée (118) conçue pour se déplacer entre une configuration pliée et une configuration moins pliée pendant l'extension locale de la gaine (100).

4. Système de gaine (1200) selon la revendication 3, dans lequel la partie pliée (118) comprend une première région pliée et une seconde région pliée et une partie de chevauchement (120) s'étendant entre les première et seconde régions pliées et
dans lequel la première région pliée est conçue pour se rapprocher de la seconde région pliée afin de raccourcir la partie de chevauchement (120) au niveau d'un emplacement axial local pendant l'application d'une force radiale vers l'extérieur par le passage du dispositif médical et dans lequel le raccourcissement de la partie de chevauchement (120) correspond à une expansion locale de la lumière (116).

5. Système de gaine (1200) selon la revendication 4, dans lequel la première région pliée est conçue pour s'éloigner davantage de la seconde région pliée afin d'allonger la partie de chevauchement (120) au niveau de l'emplacement axial local après le retrait de la force radiale vers l'extérieur et dans lequel l'allongement de la partie de chevauchement (120) correspond à une contraction locale de la lumière (116).

6. Système de gaine (1200) selon l'une quelconque des revendications 4 à 5, dans lequel la première région pliée et la seconde région pliée sont espacées de manière circonférentielle l'une de l'autre et
dans lequel la partie de chevauchement (120) s'étend de manière circonférentielle entre les première et seconde régions pliées.

7. Système de gaine selon l'une quelconque des revendications 4 à 6, dans lequel la couche interne (108) définit une couche circonférentiellement continue et la partie de chevauchement est radialement espacée d'une surface externe d'une partie non chevauchante de la couche interne (108) et
dans lequel la couche externe (110) définit une couche externe discontinue (110) comprenant une partie sous-jacente (122) écartant radialement la partie de chevauchement (120) de la surface externe de la partie non chevauchante.

8. Système de gaine (1200) selon l'une quelconque des revendications 1 à 7, dans lequel l'extrémité proximale (1212) du joint d'étanchéité (1202) définit un diamètre interne proximal et l'extrémité distale (1214) du joint d'étanchéité (1202) définit un diamètre interne distal et
dans lequel le diamètre interne proximal et le diamètre interne distal sont chacun dimensionnés et conçus pour accepter la gaine (100) dans la lumière (1216) du joint d'étanchéité (1202).

9. Système de gaine (1200) selon l'une quelconque des revendications 1 à 8, dans lequel l'extrémité proximale (1212) définit un diamètre externe proximal et dans lequel l'extrémité distale (1214) définit un diamètre externe distal, le diamètre externe proximal étant supérieur au diamètre externe distal.

10. Système de gaine (1200) selon la revendication 9, dans lequel le diamètre externe distal est dimensionné et conçu pour être logé au moins partiellement dans une lumière interne d'un dispositif de fermeture de vaisseau sanguin.

11. Système de gaine (1200) selon l'une quelconque des revendications 9 à 10, dans lequel le diamètre externe proximal définit une surface évasée.

12. Système de gaine (1200) selon la revendication 11, dans lequel la surface évasée est dimensionnée et conçue pour fournir une surface de préhension.

13. Système de gaine (1200) selon l'une quelconque des revendications 9 à 12, dans lequel le diamètre externe distal définit une surface conique.

14. Système de gaine (1200) selon la revendication 13, dans lequel la conicité est dimensionnée et conçue pour se loger au moins partiellement dans un vaisseau sanguin.

15. Système de gaine (1200) selon l'une quelconque des revendications 1 à 14, dans lequel le joint d'étanchéité est constitué d'un matériau élastomère tel que le caoutchouc de silicone, le caoutchouc nitrite, le polyisoprène, le néoprène ou le santoprène, de préférence le caoutchouc de silicone LIM6050.
